# EUROPEAN PATENT APPLICATION

(11) **EP 3 581 565 A1**
(43) Date of publication of application: **18.12.2019**
(21) Application number: 18177725.1
(22) Date of filing: 14.06.2018
(51) Int. Cl.: C07D 213/64, A61K 31/085, A61K 31/09, A61K 31/10, A61K 31/136, A61K 31/137, A61K 31/44, C07C 201/12, C07C 43/225, C07C 205/37, C07C 43/23, C07C 209/78, C07C 211/53, C07C 213/06, C07C 319/14, C07C 217/86, C07C 323/20, C07D 213/65, A61P 25/28, A61K 49/00, A61K 51/04

(54) **PHENYL BENZYL ETHER DERIVATIVE AND PREPARATION METHOD AND APPLICATION THEREOF**

(71) Applicant: Beijing Zhibo Bio-Medical Technology Co., Ltd., Beijing 102502 (CN); Zhang, Zhiyong, Beijing 102502 (CN)
(72) Inventor: CUI, Mengchao, Beijing 102502 (CN); LIN, Chunping, Beijing 102502 (CN); LIU, Boli, Beijing 102502 (CN); GUO, Yuzhi, Beijing 102502 (CN); ZHANG, Zhiyong, Beijing 102502 (CN)
(74) Representative: Müller Hoffmann & Partner

(57) **Abstract**

The present invention relates to a phenyl benzyl ether derivative, a preparation method thereof and application of the phenyl benzyl ether derivative to the preparation of medicine for diagnosing and treating the Alzheimer's disease (AD), wherein part of compounds, after being labeled by radionuclide, of the phenyl benzyl ether derivative, are used as Aβ plaque imaging agent. The structural formula of the phenyl benzyl ether derivative is shown by formula (I). The present invention develops a kind of brand new phenyl benzyl ether derivative which has high affinity with Aβ plaques in brains of AD patients. The chemical structure of the phenyl benzyl ether derivative is different from that of compounds disclosed in the prior art and the phenyl benzyl ether derivative belongs to a brand new compound for diagnosing and treating AD. The obtained Aβ plaque imaging agent has the advantages that the in-vivo stability is good, the fat solubility is low, the removal speed for the brain is fast, the problem of removing the radionuclide in vivo does not exist, and the application prospect and the market value are great.

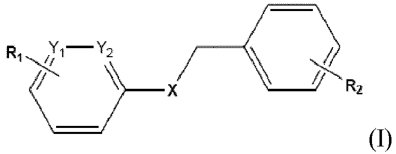

## Description

### BACKGROUND OF THE PRESENT INVENTION

### Field of Invention

The present invention relates to a field of medicine compounds, in particular to a phenyl benzyl ether derivative, a preparation method thereof and application of the phenyl benzyl ether derivative to preparation of medicine for diagnosing and treating an Alzheimer's disease (AD), wherein parts of compounds, after being labeled by radionuclide, of the phenyl benzyl ether derivative, are used as an Aβ plaque imaging agent.

### Description of Related Arts

Alzheimer's disease (AD) is a progressively developed lethal neurodegenerative disease, which is clinically featured by decrease in cognition and memory functions and decrease in daily living ability and is accompanied by various neuropsychiatric symptoms and behavior disorders. The AD mostly strikes old people. According to statistical information, the AD prevalence rate of people at age above 65 years in China is averagely 6.6%, and it has become one of major diseases after tumors, heart diseases and stroke, which seriously threatenthe physical and psychological health of the old people. At present, China is quickly stepping into an aging society. It is predicted that, by the year 2030, the proportion of population at age above 65 in China will exceed that in Japan and China will become a country with the highest degree of population aging in the world. At present, one AD patient is newly added every seven seconds around the world and 4.6 million new cases occur each year; and it is predicted that, by the year 2050, the number will exceed a hundred million. As you see, the task of AD prevention and control is difficult. Therefore, it has a very great significance to carry studies on the early-stage diagnosis method and the treatment medicine of the AD.

According to studies, senile plaques (SPs) deposited outside nerve cells in the brain due to the AD and nerve fiber tangles (NFTs) inside the nerve cells are two major pathological features. Moreover, deposition of Aβ plaques in the brain has already started 10-20 years before the AD comes on (Braak, H et. al. Acta Neuropathol., 1991, 82: 239-259).Therefore, by using the Aβ plaques in the brain as targets, developing molecular probes which have high affinity and selectivity with the Aβ plaques and adopt ingnuclear medicine imaging techniques such as single photon emission computed tomography (SPECT) and positron emission tomography (PET), the AD can be non-invasively diagnosed at an early stage from the molecular level (Cai L S, et. al. Curr Med Chem., 2007, 14: 19-52).

In the past studies, the Aβ plaque imaging agent for nuclear medicine PET imaging is developed quickly. By modifying two kinds of dyestuffs, i.e., thioflavin-T (ThT) and Congo red (CR), a wide variety of molecules have already entered a clinical test stage. For example, 2-phenylbenzothiazolerepresentative compound [¹¹C]PIB (Pittsburgh compound B) is the most commonly used Aβ plaque imaging agent at present (Klunk W E, et. al. Annals of Neurology., 2004, 55: 306-319), and since the analogue [¹⁸F] thereof is labeled by adopting ₁₈F, the clinical application prospect of the analogue [¹⁸F] may be greater (Koole M, et. al. J. Nucl. Med., 2009, 50: 818-22); and for diphenylethene derivative [¹⁸F] BAY94-9172, phase-III clinical tests are being actively carried out now (Rowe C C, et. al. Lancet Neurol., 2008, 7:129-35), and [¹⁸F] AV-45 has already been approved by FDA of the United States.

However, no breakthrough has made in the Aβ plaque imaging agent for SPECT imaging, wherein 2-phenyl imidazopyridine derivative [¹²³I] IMPY is the first SPECT imaging agent which enters the clinical stage (Newberg. A B, et. al. J. Nucl. Med. 2006, 47: 748-754). However, due to the in-vivo stability thereof is poor, it is weeded out quickly. Other Aβ plaque imaging agents which are labeled by radioactive iodine have the common disadvantages of high fat solubility, slow brain removing speed and in-vivo radioactive iodine removal.

Therefore, ifa kind of brand new compounds which have high affinity with the Aβ plaques in the brain can be developed and are subjected to radionuclide labeling, an Aβ plaque imaging agent which can be used for the early-stage diagnosis of the AD can be obtained and the application prospect and the economic value will be great without any doubt.

### SUMMARY OF THE PRESENT INVENTION

In order to solve the above-mentioned technical problems, one purpose of the present invention is to provide a phenyl benzyl ether derivative. Compounds thereof have high affinity with Aβ plaques in brains of AD patients and belong to brand new compounds for diagnosing and treating the AD.

Another purpose of the present invention is to provide a preparation method of the phenyl benzyl ether derivative.

Another purpose of the present invention is to provide an Aβ plaque imaging agent prepared by using the phenyl benzyl ether derivative.

Another purpose of the present invention is to provide application of the Aβ plaque imaging agent to preparation of a medicine for diagnosing an amyloidosis disease, including early-stage diagnosis of the AD.

Another purpose of the present invention is to provide application of the phenyl benzyl ether derivative to preparation of a medicine for diagnosing and treating the AD.

In order to realize the above-mentioned purposes, a phenyl benzyl ether derivative is provided by the present invention, wherein a structural formula thereof is shown by a formula (I):
wherein X is O, NH or S; Y₁ and Y₂ respectively and independently denote-CH- or nitrogen;
R₁ and R₂ respectively and independently denote nitrogen, halogen, hydroxyl, hydrosulfuryl, alkoxy, alkyl, carbocyclic alkyl, heterocyclic alkyl, nitro, amino, alkylamino, cyan, carboxyl, aryl, heteraryl, arylalkoxy, substituted arylalkoxy, aryloxy, substituted aryloxy, arylalkenyl, substituted arylalkenyl, -O (CH₂)mNRaRb, -CO-NRaRb, -NHCO-Ra, -Sn(alkyl)₃, -(CH₂)m-Z, -O (CH₂)m-Z, -(CH₂)m- aryl or -(OCH₂CH₂)n-Z;
wherein Ra and Rb respectively and independently denote hydrogen, akyl or-(CH₂)m- aryl;
wherein Z denotes halogen, hydroxyl, trifluoromethylsulfonyl, methylsulfonyl or tolylsulfonyl;
wherein the above-mentioned any m and n are respectively integers from 1 to 6, preferably integers from 1 to 3 respectively.

More preferably, Y₁ and Y₂ are simultaneously -CH- or nitrogen.

More preferably, Y₁ and Y₂ are respectively -CH- or nitrogen, or Y₁ and Y₂ are respectively nitrogen or -CH-.

More preferably,-(CH₂)m-aryl is -(CH₂)m-phenyl, including -(CH₂)m-aryl denoted by R₁, R₂, Ra and Rb.

Preferably, R₁ and R₂ are o-substituents, m-substituents or p-substituents.

Preferably, the halogen is fluorine, chlorine, bromine or iodine, including halogen denoted by R₁ and R₂ and halogen denoted by Z.

Preferably, the alkoxy is C₁-C₁₂ alkoxy, preferably C₁-C₆ alkoxy.

More preferably, the alkoxy is methoxy, ethyoxyl, propoxy or butoxy.

Preferably, the alkyl is C₁-C₁₂ alkyl, preferably C₁-C₆ alkyl.

More preferably, the alkyl is methyl, ethyl, propyl, isopropyl or tert-butyl.

Preferably, the carbocyclic alkyl is three-membered to six-membered carbocyclic alkyl.

More preferably, the carbocyclic alkyl is cyclopropyl, cyclopentyl or cyclohexyl.

Preferably, the heterocyclic alkyl is three-membered to six-membered heterocyclic alkyl.

More preferably, the heterocyclic alkyl is piperidyl, piperazinyl or morpholine cyclic group.

Preferably, the alkylamino is C₁-C₁₂ alkylamino, preferably C₁-C₆ alkylamino.

More preferably, the alkylamino is N-methylamino, dimethylamino, diethylamino, dipropylamino or diisopropylamino.

Preferably, the aryl is phenyl or naphthyl.

Preferably, the heteraryl is pyridyl, furyl, thienyl, benzothiazolyl, benzofuryl or benzoxazolyl.

Preferably, the arylalkoxy is C₅-C₇ aryl C₁-C₁₂ alkoxy.

More preferably, the arylalkoxy is phenylmethoxyl or phenylethyoxyl.

Preferably, the substituted arylalkoxy is substituted C₅-C₇ aryl C₁-C₁₂ alkoxy.

More preferably, the substituted arylalkoxy is substituted phenylmethoxyl or substituted phenylethyoxyl.

Preferably, the aryloxy is C₅-C₇ aryloxy.

More preferably, the aryloxy is cyclopentadienyloxy or phenyloxy.

Preferably, the substituted aryloxy is substituted C₅-C₇ aryloxy.

More preferably, the substituted aryloxy is substituted cyclopentadienyloxy or substituted phenyloxy.

Preferably, the arylalkenyl is C₅-C₇ aryl C₂-C₆ alkenyl.

More preferably, the arylalkenyl is phenylvinyl.

Preferably, the substituted arylalkenyl is substituted C₅-C₇ aryl C₂-C₆ alkenyl.

Moreover preferably, the substituted arylalkenyl is substituted phenylvinyl.

More preferably, the aryl in the substituted arylalkoxy is substituted by halogen, hydroxyl, alkoxy, nitro, amino or alkylamino.

More preferably, the aryl in the substituted aryloxy is substituted by halogen, hydroxyl, alkoxy, nitro, amino or alkylamino.

More preferably, the aryl in the substituted aryalkenyl is substituted by halogen, hydroxyl, alkoxy, nitro, amino or alkylamino.

More preferably, the halogen in the substituted arylalkoxy, the substituted aryloxy and the substituted arylalkenyl is fluorine, chlorine, bromine or iodine, wherein the alkoxy is methoxy, ethyoxyl, propoxy or butoxy; wherein the alkylamino is N-methylamino, dimethylamino, diethylamino, dipropylamino or diisopropylamino.

The phenyl benzyl ether derivative is provided by the present invention, wherein more preferably, the structural formula thereof is shown by a formula (I-1): 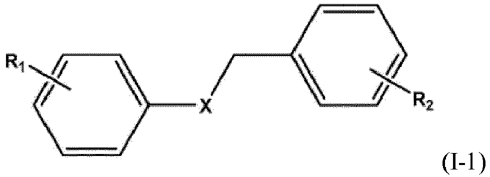
wherein X is O, NH or S;
R₁ is nitro, methoxy, hydroxyl, fluorine, chorine, bromine, iodine, hydrogen, tert-butyl, amino, methylamino, dimethylamino, -OCH₂CH₂F, -Sn(butyl)₃, -OCH₂CH₂OH, -(OCH₂CH₂)₃OH, -OCH₂CH₂OTs, -(OCH₂CH₂)₃OTs or -(OCH₂CH₂)₃F;
R₂ is iodine, methoxy, bromine, hydrogen, -OCH₂CH₂F, -Sn(butyl)₃,-OCH₂CH₂Br, -OCH₂CH₂OTs or dimethylamino.

More preferably, R₁ and R₂ are respectively:

| R₁ | R₂ |
|---|---|
| Nitro | Iodine |
| Methoxy | Iodine |
| Hydroxyl | Iodine |
| Fluorine | Iodine |
| Chlorine | Iodine |
| Bromine | Iodine |
| Iodine | Iodine |
| Hydrogen | Iodine |
| Tert-butyl | Iodine |
| Amino | Iodine |
| Methylamino | Iodine |
| Dimethylamino | Iodine |
| Iodine | Methoxy |
| Methoxy | Bromine |
| Bromine | Methoxy |
| Dimethylamino | Bromine |
| -OCH₂CH₂F | Hydrogen |
| -OCH₂CH₂F | Iodine |
| -OCH₂CH₂F | Bromine |
| Iodine | -OCH₂CH₂F |
| Bromine | -OCH₂CH₂F |
| Methoxy | -Sn(butyl)₃ |
| -Sn(butyl)₃ | Methoxy |
| Dimethylamino | -Sn(butyl)₃ |
| -OCH₂CH₂F | -Sn(butyl)₃ |
| -Sn(butyl)₃ | -OCH₂CH₂F |
| Methoxy | -OCH₂CH₂F |
| Methoxy | -OCH₂CH₂Br |
| Nitro | -OCH₂CH₂F |
| Amino | -OCH₂CH₂F |
| Methylamino | -OCH₂CH₂F |
| Dimethylamino | -OCH₂CH₂F |
| Methoxy | -OCH₂CH₂OTs |
| -OCH₂CH₂OH | Hydrogen |
| -(OCH₂CH₂)₃OH | Hydrogen |
| -OCH₂CH₂OH | Methoxy |
| -(OCH₂CH₂)₃OH | Methoxy |
| -OCH₂CH₂OTs | Methoxy |
| -(OCH₂CH₂)₃OTs | Methoxy |
| -OCH₂CH₂F | Methoxy |
| -(OCH₂CH₂)₃F | Methoxy |
| Iodine | Dimethylamino |

Preferably, R₁ and R₂ are o-substituents, m-substituents or p-substituents.

More preferably, R₁, R₂ and X are respectively:

| R₁ | R₂ | X |
|---|---|---|
| p-OMe | I | O |
| m-OMe | I | O |
| o-OMe | I | O |
| p-OH | I | O |
| m-OH | I | O |
| o-OH | I | O |
| p-F | I | O |
| p-Cl | I | O |
| p-Br | I | O |
| p-I | I | O |
| P-H | I | O |
| p-Bu^{t} | I | O |
| p-NH₂ | I | O |
| m-NH₂ | I | O |
| o-NH₂ | I | O |
| p-NHMe | I | O |
| m-NHMe | I | O |
| o-NHMe | I | O |
| p-NMe₂ | I | O |
| m-NMe2 | I | O |
| I | P-OMe | O |
| p-OMe | I | S |
| p-OCH₂CH₂F | I | O |
| p-I | OCH₂CH₂F | O |
| p-OMe | OCH₂CH₂F | O |
| p-NHMe | OCH₂CH₂F | O |
| p-NMe₂ | OCH₂CH₂F | O |
| p-OCH₂CH₂F | OMe | O |
| P-(OCH₂CH₂)₃F | OMe | O |
| p-I | NMe₂ | NH |

The phenyl benzyl ether derivative is provided by the present invention, wherein more preferably, the structural formula thereof is shown by a formula (I-2): 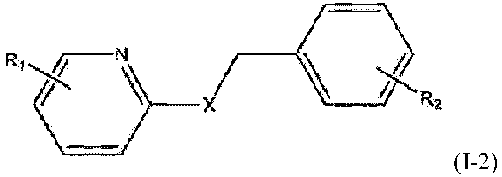
wherein X is O, NH or S;
R₁ is hydrogen, bromine, iodine, nitro, amino, methylamino or dimethylamino;
R₂ is iodine, methoxy or -OCH₂CH₂F;

More preferably, R₁ and R₂ are respectively:

| R₁ | R₂ |
|---|---|
| Hydrogen | Iodine |
| Bromine | Iodine |
| Iodine | Iodine |
| Nitro | Iodine |
| Amino | Iodine |
| Methylamino | Iodine |
| Dimethylamino | Iodine |
| Iodine | Methoxy |
| Iodine | -OCH₂CH₂F |

wherein R₁ and R₂ are o-substituents, m-substituents or p-substituents.

More preferably, R₁ and R₂ are respectively:

| R₁ | R₂ | X |
|---|---|---|
| p-H | I | O |
| p-I | I | O |

The phenyl benzyl ether derivative is provided by the present invention, wherein more preferably, the structural formula thereof is shown by formula (I-3): 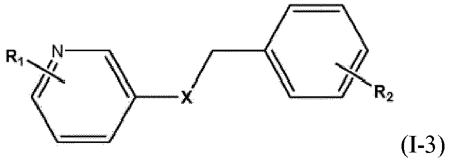
wherein X is O, NH or S;
R₁ is chlorine, bromine or iodine; and R₂ is iodine, methoxy or -OCH₂CH₂.

More preferably, R₁ and R₂ are respectively:

| R₁ | R₂ |
|---|---|
| Chlorine | Iodine |
| Bromine | Iodine |
| Iodine | Iodine |
| Iodine | Methoxy |
| Iodine | -OCH₂CH₂F |

wherein R₁ and R₂ are o-substituents, m-substituents or p-substituents.

More preferably, R₁, R₂ and X are respectively:

| R₁ | R₂ | X |
|---|---|---|
| p-Cl | I | O |
| p-Br | I | O |
| p-I | I | O |
| p-I | OCH₂CH₂F | O |

The phenyl benzyl ether derivative is provided by the present invention, wherein more preferably, when the phenyl benzyl ether derivative contains fluorine atoms, F is ¹⁸F or ¹⁹F; when the phenyl benzyl ether derivative contains iodine atoms, I is ¹²³I, ¹²⁴I, ¹²⁵I, ¹²⁷I or ¹³¹I; and when the phenyl benzyl ether derivative contains methyl, methoxy, N-methylamino or dimethylamino, -CH₃ is -¹¹CH3, -OCH₃ is -O¹¹CH₃,-NHCH₃ is -NH¹¹CH₃, and -N(CH₃)₂ is -N(¹¹CH₃)₂ or -N(CH₃)(¹¹CH₃).

More preferably, when F is ¹⁸F or I is ¹²⁴I, the obtained phenyl benzyl ether derivative is able to be used as the Aβ plaque imaging agent, especially a PET Aβ plaque imaging agent.

More preferably, when -CH₃ is -¹¹CH3, -OCH₃ is -O¹¹CH₃, -NHCH₃ is-NH¹¹CH₃ and -N(CH₃)₂ is -N(¹¹CH₃)₂ or -N(CH₃)(¹¹CH₃), the obtained phenyl benzyl ether derivative is able to be used as the Aβ plaque imaging agent, especially the PET Aβ plaque imaging agent.

More preferably, when I is ¹²⁵I, ¹²³I or ¹³¹I, the obtained phenyl benzyl ether derivative is able to be used as the Aβ plaque imaging agent, especially a SPECT Aβ plaque imaging agent.

The phenyl benzyl ether derivative is provided by the present invention, wherein more preferably, when the phenyl benzyl ether derivative simultaneously contains two and more than two substituents of fluorine, iodine, methyl, methoxy, N-methylamino and dimethylamino, only one substituent is prepared into the above-mentioned corresponding radionuclide to obtain the Aβ plaque imaging agent.

A preparation method of the phenyl benzyl ether derivative is provided by the present invention, wherein a reaction equation is: 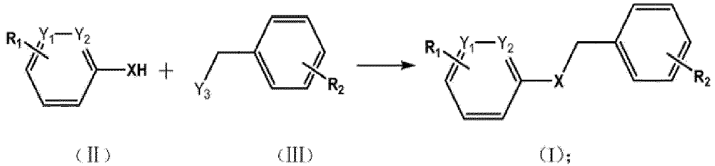 wherein Y₃ is bromine or chlorine; and R₁, R₂, X, Y₁ and Y₂ are correspondingly defined as that in the phenyl benzyl ether derivative shown by the structural formula such as the formula (I).

Another preparation method of the phenyl benzyl ether derivative provided by the present invention, wherein a reaction equation is: 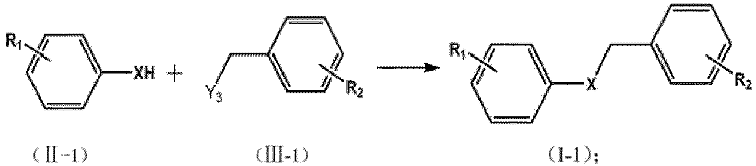 wherein Y₃ is bromine or chlorine; R₁, R₂ and X are correspondingly defined as that in the phenyl benzyl ether derivative shown by the structural formula such as the formula (I-1).

Another preparation method of the phenyl benzyl ether derivative provided by the present invention, wherein a reaction equation is: 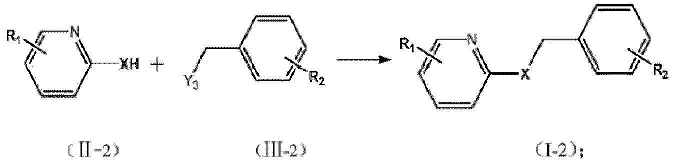 wherein Y₃ is bromine or chlorine; R₁, R₂ and X are correspondingly defined as that in the phenyl benzyl ether derivative shown by the structural formula such as the formula (I-2).

Another preparation method of the phenyl benzyl ether derivative provided by the present invention, wherein a reaction equation is: 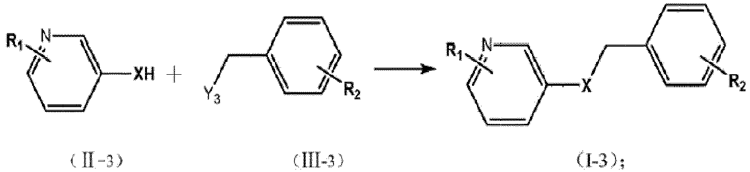 wherein Y₃ is bromine or chlorine; R₁, R₂ and X are correspondingly defined as that in the phenyl benzyl ether derivative shown by the structural formula such as the formula (I-3).

The present invention further provides an Aβ plaque imaging agent prepared by using the phenyl benzyl ether derivative.
wherein, when the phenyl benzyl ether derivative contains fluorine atoms, the prepared compounds containing radionuclide F-18 are used as the Aβ plaque imaging agent, especially the PET Aβ plaque imaging agent.

Or when the phenyl benzyl ether derivative contains iodine atoms, the prepared compounds containing radionuclide I-124 are used as the Aβ plaque imaging agent, especially the PET Aβ plaque imaging agent.

Or when the phenyl benzyl ether derivative contains methyl, methoxy, N-methylamino or dimethylamino, the prepared compounds containing radionuclide C-11(-¹¹CH₃, -O¹¹CH₃, -NH¹¹CH₃ -N(^{N}CH₃)₂ or -N(CH₃)(¹¹CH₃)) are used as the Aβ plaque imaging agent, especially the PET Aβ plaque imaging agent.

Or when the phenyl benzyl ether derivative contains iodine atoms, the prepared compounds containing radionuclide I-123, I-125 or I-131 are used as the Aβ plaque imaging agent, especially the SPECT Aβ plaque imaging agent.

More preferably, when the phenyl benzyl ether derivative simultaneously contains two and more than two substituents of fluorine, iodine, methyl, methoxy, N-methylamino and dimethylamino, only one substituent is prepared into the above-mentioned corresponding radionuclide to obtain the Aβ plaque imaging agent.

Preferably, the Aβ plaque imaging agent is a single photon or positron Aβ plaque imaging agent.

Preferably, the Aβ plaque imaging agent is able to be used for PET imaging or SPECT imaging.

The Aβ plaque imaging agent provided by the present invention is able to be used for the early-stage diagnosis of amyloidosis in the AD.

The present invention further provides application of the phenyl benzyl ether derivative to the preparation of the medicine for diagnosing and treating the AD.

The present invention prepares a kind of phenyl benzyl ether compounds with brand new structures. According to in-vitro competitive binding experiments, this kind of molecules has very high affinity with Aβ₁₋₄₂ aggregates; according to in-vitro autoradiography experiments, this kind of molecules labeled by I-125 or F-18 can be specifically bound with Aβ plaques in AD human brain sections or brains of AD transgenic mice; according to in-vivo biological distribution experiments carried to normal mice, part of imaging agent labeled by I-125 or F-18 has the advantages of high initial brain intake and fast removing speed; and it is expected to become a new single photon or positron Aβ plaque imaging agent for clinical imaging.

In conclusion, the present invention develops a kind of brand new phenyl benzyl ether derivatives which have high affinity with Aβ plaques in brains of AD patients, the chemical structures are different from that of the compounds disclosed by the prior art, especially thioflavin-T and Congo red, and the brand new phenyl benzyl ether derivatives belong to brand new compounds for diagnosing and treating the AD; and the obtained Aβ plaque imaging agent has the advantages that the in-vivo stability is good, the fat solubility is low, the brain removing speed is fast, the problem of removing the radionuclide in vivo does not exist, and the application prospect and the market value are great.

### BRIEF DESCRIPTION OF THE DRAWINGS

Fig.1 is a reaction route diagram of a part 1 of an embodiment 1 and an embodiment 5.
Fig.2 is a reaction route diagram of a part 2 of the embodiment 1 and the embodiment 5.
Fig.3 is a reaction route diagram of an embodiment 3.
Fig.4 is a reaction route diagram of an embodiment 4.
Fig.5 and Fig.6 are display images of autoradiography experiments in an embodiment 2.

### DETAILED DESCRIPTION OF THE PREFERRED EMBODIMENT

The following embodiments are used for describing the present invention instead of limiting the scope of the present invention.

Operations which are not mentioned in the present invention are conventional operations in the field.

[¹²⁵I] NaI solution used in the present invention is bought from China Isotope & Radiation Corporation.

Other materials used in the present invention are conventional materials which can be bought from the market or can be prepared by adopting the existing methods (for example, 4-hydroxy-N, N-dimethylaniline).

A Room temperature in the present invention is 25 °C.

Concentration (%) of each material in the present invention is mass concentration.

### Embodiment 1: synthesis of iodo compounds

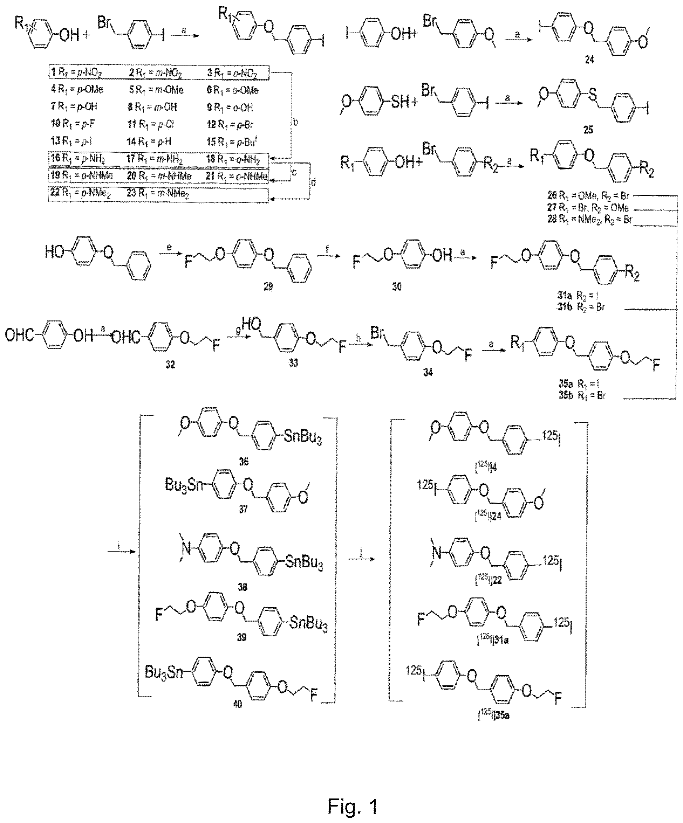

A synthesis reaction route is shown in Fig.1. Serial numbers of compounds in the embodiment 1 are consistent with serial numbers in the reaction route in Fig.1.

In the synthesis route shown in Fig.1, reagents and conditions are as follows: (a) K₂CO₃, DMF, 90 °C; (b)SnCl₂•2H₂O, EtOH, HCl, reflux; (c) 1: NaoMe, (CH₂O)ₙ, MeOH, reflux; 2: NaBH₄, reflux; (d) (CH₂O)ₙ, NaBH₃CN, HAc, r. t.; (e) 1-bromo-fluoroethane, KOH, ethanol, reflux; (f)10% Pd/C, latmH₂, 50 °C; (g) NaBH₄, MeOH, 0 °C; (h) PBr₃, CH₂Cl₂, r. t.; (i) (Bu₃Sn)₂, (PPh₃)₄Pd, methylbenzene, Et₃N, reflux; (j) [¹²⁵I] NaI, HCl (1M), H₂O₂ (3%).

### 1) Synthesis of 1-iodo-4-((4-nitrophenoxy) methyl) benzene (a compound 1)

4-nitrophenol (2.78g, 20mmol) and 4-iodobenzyl bromide (5.00g, 20mmol) are dissolved in 5ml of anhydrous DMF, and K₂CO₃ (5.53g, 40mmol) is added. Reaction is conducted for 5h at 90 °C under a reflux and stirring state, reaction is monitored through TLC till completion, depressurization is conducted to remove solvent, 50ml of deionized water is added, white precipitate is separated out, suction filtration is conducted, the precipitate is washed with water and methanol recrystallization is conducted to obtain white solid 1 (1.69g, 95.2%). HPLC: 7.51min, 99.8%; mp 147.4-148.1 °C; ¹H NMR (400MHz, CDCl₃) δ 8.21 (d, J=9.2Hz, 2H), 7.75 (d, J=8.3Hz, 2H), 7.18 (d, J=8.3Hz, 2H), 7.01 (d, J=9.2Hz, 2H), 5.10 (s, 2H).

### 2) Synthesis of 1-(4-iodobenzyloxy)-3-nitrobenzene (a compound 2)

Preparation is conducted according to the preparation method of the compound 1 (reactant 4-nitrophenol is replaced by 3-nitrophenol) to obtain white solid 2 (345.5mg, 97.3%). HPLC: 8.53min, 99.9%; mp 75.2-75.9 °C; ¹H NMR (400MHz, CDCl₃) δ 7.85 (d, J=8.0Hz, 1H), 7.80 (s, 1H), 7.74 (d, J=8.2Hz, 2H), 7.45 (t, J=8.2Hz, 1H), 7.30-7.26 (m, 1H), 7.19 (d, J=8.1Hz, 2H), 5.09 (s, 2H).

### 3) Synthesis of 1-(4-iodobenzyloxy)-2-nitrobenzene (a compound 3)

Preparation is conducted according to the preparation method of the compound 1 (reactant 4-nitrophenol is replaced by 2-nitrophenol) to obtain yellow solid 3 (238.9mg, 67.3%). HPLC: 6.03min, 99.5%; mp 70.6-71.5 °C; ¹H NMR (400MHz, CDCl₃) δ 7.87 (dd, J=8.1, 1.4Hz, 1H), 7.73 (d, J=8.2Hz, 2H), 7.56-7.46 (m, 1H), 7.22 (d, J=8.1Hz, 2H), 7.09-7.04 (m, 2H), 5.18 (s, 2H).

### 4) Synthesis of 1-iodo-4-((4-methoxyphenoxy) methyl) benzene (a compound 4)

Preparation is conducted according to the preparation method of the compound 1 (reactant 4-nitrophenol is replaced by 4-methoxyphenol) to obtain white solid 4 (255.3mg, 75.1%). HPLC: 8.28min, 99.5%; mp 128.9-130.2 °C; ¹H NMR (400MHz, CDCl₃) δ 7.71 (d, J=8.2Hz, 2H), 7.17 (d, J=8.2Hz, 2H), 6. 89 (d, J=9.2Hz, 2H), 6.83 (d, J=9. 2Hz, 2H), 4.96 (s, 2H), 3.77 (s, 3H). ¹³C NMR (101MHz, CDCl₃) δ 154.18, 152.68, 137.63, 137.08, 129.25, 115.92, 114.72, 93.30, 70.07, 55.72.

### 5) Synthesis of 1-(4-iodobenzyloxy)-3-methoxybenzene (a compound 5)

Preparation is conducted according to the preparation method of the compound 1 (reactant 4-nitrophenol is replaced by 3-methoxyphenol) to obtain white solid 5 (211.7mg, 62.2%). HPLC: 8.68min, 98.5%; mp 77.4-78.7 °C; ¹H NMR (400MHz, CDCl₃) δ 7.71 (d, J=8.1Hz, 2H), 7.21-7.17 (m, 3H), 6.57-6.50 (m, 2H), 4.99 (s, 2H), 3.79 (s, 3H).

### 6) Synthesis of 1-(4-iodobenzyloxy)-2-methoxybenzene (a compound 6)

Preparation is conducted according to the preparation method of the compound 1 (reactant 4-nitrophenol is replaced by 2-methoxyphenol) to obtain white solid 6 (288.1mg, 84.7%). HPLC: 6.63min, 98.7%; mp 110.4-110.8 °C; ¹H NMR (400MHz, CDCl₃) 87.69 (d, J=8.1Hz, 2H), 7.19 (d, J=8.0Hz, 2H), 6.97-6.91 (m, 2H), 6.85 (d, J=3.8Hz, 2H), 5.10 (s, 2H), 3.89 (s, 3H).

### 7) Synthesis of 4-(4-iodobenzyloxy) phenol (a compound 7)

P-dihydroxybenzene (110.1mg, 1.0mmol) and 4-iodobenzyl bromide (296.9g, 1.0mmol) are dissolved in 5ml of anhydrous DMF, and K₂CO₃ (276.4mg, 2.0mmol) is added. Reaction is conducted for 2h at 90 °C under a reflux and stirring state, reaction is monitored through TLC till completion, depressurization is conducted to remove solvent, 50ml of deionized water is added, extraction is conducted by using CH₂Cl₂ (3x10ml), organic phases are combined, drying is conducted by using anhydrous MgSO₄, suction filtration is conducted, depressurization is conducted to remove solvent, and residues are separated through silica gel column chromatography (petroleum ether/ethyl acetate=4/1) to obtain white solid 7 (49.3mg, 15.1%). HPLC: 3.75min, 99.3%; mp 152.2-153.7 °C; ¹H NMR (400MHz, CDCl₃) δ 7.71 (d, J=8.3Hz, 2H), 7.17(d, J=8.2Hz, 2H), 6.85-6.81 (m, 2H), 6.78- 6.74 (m, 2H), 4.95 (s, 2H), 4.41 (s, 1H).

### 8) Synthesis of 3-(4-iodobenzyloxy) phenol (a compound 8)

Preparation is conducted according to the preparation method of the compound 7 (reactant p-dihydroxybenzene is replaced by m-dihydroxybenzene) to obtain white solid 8(93.1mg, 28.5%). HPLC: 3.95min, 98.6%; mp 109.9-110.6 °C; ¹H NMR (400MHz, CDCl₃) δ 7.71 (d, J=7.9Hz, 2H), 7.17 (d, J=8.2Hz, 2H),7.16-7.09 (m, 1H), 6.54 (d, J=8.6Hz, 1H), 6.46- 6.44 (m, 2H), 4.98 (s, 2H), 4.74 (s, 1H).

### 9) Synthesis of 2-(4-iodobenzyloxy) phenol (a compound 9)

Preparation is conducted according to the preparation method of the compound 7 (reactant p-dihydroxybenzene is replaced by o-dihydroxybenzene) to obtain white solid 9 (109.8mg, 33.7%). HPLC: 4.25min, 99.9%; mp 62.1-63.4 °C; ¹H NMR (400MHz, CDCl₃) δ 7.74 (d, J=8.2Hz, 2H), 7.17 (d, J=8.0Hz, 2H), 6.98-6.95 (m, 1H), 6.93-6.88 (m, 2H), 6.85- 6.81(m, 1H), 5.62 (s, 1H), 5.06 (s, 2H).

### 10) Synthesis of 1-fluoro-4-(4-iodobenzyloxy) benzene (a compound 10)

Preparation is conducted according to the preparation method of the compound 1 (reactant 4-nitrophenol is replaced by 4-fluorophenol) to obtain white solid 10 (253.1mg, 77.1%). HPLC: 8.92min, 98.0%; mp 62.3-62.8 °C; ¹H NMR (400MHz, CDCl₃) δ 7.72 (d, J = 8.2Hz, 2H), 7.17 (d, J = 8.2Hz, 2H), 7.00-6.95 (m, 2H), 6.91- 6.85 (m, 2H), 4.97 (s, 2H).[0148]

### 11) Synthesis of 1-chloro-4-(4-iodobenzyloxy) benzene (a compound 11)

Preparation is conducted according to the preparation method of the compound 1 (reactant 4-nitrophenol is replaced by 4-chlorophenol) to obtain white solid 11 (136.2mg, 79.0%). HPLC: 12.86min, 99.6%; mp 107.5-108.1 °C; ¹H NMR (400MHz, CDCl₃) δ 7.72 (d, J=8.2Hz, 2H), 7.24 (d, J=8.9Hz, 2H), 7.16 (d, J=8.2Hz, 2H), 6.87 (d, J=8.9Hz, 2H), 4.98 (s, 2H).

### 12) Synthesis of 1-bromo-4-(4-iodobenzyloxy) benzene (a compound 12)

Preparation is conducted according to the preparation method of the compound 1 (reactant 4-nitrophenol is replaced by 4-bromophenol) to obtain white solid 12 (302.5mg, 77.8%). HPLC: 14.61min, 99.3%; mp 122.6-123.5 °C; ¹H NMR (400MHz, CDCl₃) δ 7.71 (d, J=8.2Hz, 2H), 7.37 (d, J=9. 0Hz, 2H), 7.16 (d, J=8.2Hz, 2H), 6.82 (d, J=8.9Hz, 2H), 4.97 (s, 2H).

### 13) Synthesis of 1-iodo-4-(4-iodobenzyloxy) benzene (a compound 13)

Preparation is conducted according to the preparation method of the compound 1 (reactant 4-nitrophenol is replaced by 4-iodophenol) to obtain white solid 13 (436.0mg, 89.3%). HPLC: 17.49min, 98.2%; mp 135.0-135.9 °C; ¹H NMR (400MHz, CDCl₃) δ 7.71 (d, J=8.2Hz, 2H), 7.56 (d, J=8.7Hz, 2H), 7.15 (d, J=8.1Hz, 2H), 6.72 (d, J=8.8Hz, 2H), 4.97 (s, 2H).

### 14) Synthesis of 1-iodo-4-(phenoxymethyl) benzene (a compound 14)

Preparation is conducted according to the preparation method of the compound 1 (reactant 4-nitrophenol is replaced by phenol) to obtain white solid 14 (310.1mg, 72.1%). HPLC: 9.59min, 99.2%; mp 96.7-97.6 C; ¹H NMR (400MHz, CDCl₃) δ 7.72 (d, J = 8.2Hz, 2H), 7.32-7.27 (m, 2H), 7.19 (d, J = 8.1Hz, 2H), 6.99-6.94 (m, 3H), 5.02 (s, 2H).

### 15) Synthesis of 1-tert-butyl-4-(4-iodobenzyloxy) benzene (a compound 15)

Preparation is conducted according to the preparation method of the compound 1 (reactant 4-nitrophenol is replaced by 4-tert-butylphenol) to obtain white solid 15 (366.2mg, 87.4%). HPLC: 27.14min, 98.3%; mp 91.9-93.0 °C; ¹H NMR (400MHz, CDCl₃) δ 7.71 (d, J=8.2Hz, 2H, 7.31 (d, J=8.8Hz, 2H), 7.18 (d, J=8.2Hz, 2H), 6.89 (d, J=8.8Hz, 2H), 4.99 (s, 2H), 1.30 (s, 9H).

### 16) Synthesis of 4-(4-iodobenzyloxy) aniline (a compound 16)

The compound 1 (1.42g, 4.0mmol) and SnCl₂•2H₂O (1.66g, 8.0mmol) are dissolved in 25ml of ethanol, 2ml of concentrated hydrochloric acid is dripped, reaction is conducted for 2h at 80 °C under a reflux and stirring state, cooling to room temperature is conducted, 30ml of 1M NaOH water solution is added to neutralize the hydrochloric acid and precipitateSnCl₂, extraction is conducted by using ethyl acetate (3x10ml), organic phases are combined, drying is conducted by using anhydrous MgSO₄, suction filtration is conducted, depressurization is conducted to remove solvent, and residues are separated through silica gel column chromatography (petroleum ether/ethyl acetate=2/1) to obtain white solid 16 (643.3mg, 49.5%). HPLC: 4.16min, 98.4%; mp 138.6-140.0 °C; ¹H NMR (400MHz, CDCl₃) δ 7.70 (d, J=8.2Hz, 2H), 7.16 (d, J=7.9Hz, 2H), 6.78 (d, J=8.7Hz, 2H), 6.64 (d, J=8.8Hz, 2H), 4.93 (s, 2H), 3.44 (s, 2H).

### 17) Synthesis of 3-(4-iodobenzyloxy) aniline (a compound 17)

Preparation is conducted according to the preparation method of the compound 16 (reactant compound 1 is replaced by the compound 2) to obtain white solid 17 (695.5mg, 73.1%). HPLC: 4.50min, 99.4%; mp 153.9-154.8 °C; ¹H NMR (400MHz, CDCl₃) δ 7.70 (d, J = 8.1Hz, 2H), 7.17(d, J = 8. 0Hz, 2H), 7.07 (t, J = 8.0Hz, 1H), 6.40-6.34 (m, 3H), 4.97 (s, 2H).

### 18) Synthesis of 2-(4-iodobenzyloxy) aniline (a compound 18)

Preparation is conducted according to the preparation method of the compound 16 (reactant compound 1 is replaced by the compound 3) to obtain white solid 18 (99.3mg, 11.4%). HPLC: 4.88min, 99.1%; mp 99.1-100.1 °C; ¹H NMR (400MHz, CDCl₃) δ 7.71 (d, J = 8.3Hz, 2H), 7.19 (d, J = 8.3Hz, 2H), 6.85-6.78 (m, 3H), 6.75-6.71 (m, 1H), 5.03 (s, 2H).

### 19) Synthesis of 4-(4-iodobenzyloxy)-N-methylaniline (a compound 19)

The compound 16 (162.6mg, 0.5mmol) and paraformaldehyde (60.0mg, 2.0mmol) are dissolved in 30ml of anhydrous methanol, 5ml of NaOCH₃ (54.0mg, 1.0mmol) methanol solution is added drop by drop, and reaction is conducted for 2h under a reflux and stirring state. After cooling to room temperature is conducted, NaBH₄ (75.6mg, 2.0mmol) is slowly added and continuously reaction is conducted for 2h under a reflex and stirring state. Depressurization is conducted to remove solvent, 50ml of 1M NaOH solution is added, white precipitate is separated out, suction filtration is conducted, the precipitate is washed with water, and methanol recrystallization is conducted to obtain pink solid19 (152.4mg, 89.9%). HPLC: 6.34min, 99.2%; mp 93.9-95.2 °C; ¹H NMR (400MHz, CDCl₃) δ 7.70 (d, J=8. 2Hz, 2H), 7. 17 (d, J=8. 1Hz, 2H), 6.84 (d, J=8. 7Hz, 2H), 6.58 (d, J=8.5Hz, 2H), 4.94 (s, 2H), 2.81(s, 3H).

### 20) Synthesis of 3-(4-iodobenzyloxy)-N-methylaniline (a compound 20)

The compound 17 (162.6mg, 0.5mmol) and paraformaldehyde (60.0mg, 2.0mmol) are dissolved in 30ml of anhydrous methanol, 5ml of NaOCH₃ (54.0mg, 1.0mmol) methanol solution is added drop by drop, and reaction is conducted for 2h under a reflux and stirring state. After cooling to room temperature is conducted, NaBH₄ (75.6mg, 2.0mmol) is slowly added and continuously reaction is conducted for 2h under a reflex and stirring state. Depressurization is conducted to remove solvent, 50ml of 1M NaOH solution is added, extraction is conducted by using CH₂Cl₂ (3x10ml), organic phases are combined, drying is conducted by using anhydrous MgSO₄, suction filtration is conducted, depressurization is conducted to remove solvent, and residues are separated through silica gel column chromatography (petroleum ether/ethyl acetate=4/1) to obtain a colorless oily compound 20 (71.0mg, 41.8%). HPLC: 6.60min, 99.9%; mp 45.3-46.6 °C; ¹H NMR (400MHz, CDCl₃) δ 7.71 (d, J=8.1Hz, 2H), 7.19 (d, J=8.0Hz, 2H), 7.09 (t, J=8.0Hz, 1H), 6.32 (d, J=8.0Hz, 1H), 6.27 (d, J=7.9Hz, 1H), 6.24 (s, 1H), 4.99 (s, 2H), 2.83 (s, 3H).

### 21) Synthesis of 2-(4-iodobenzyloxy)-N-methylaniline (a compound 21)

Preparation is conducted according to the preparation method of the compound 19 (reactant compound 16 is replaced by the compound 18), extraction is conducted by using CH₂Cl₂ (3x10ml) after reaction is completed, organic phases are combined, drying is conducted by using anhydrous MgSO₄, suction filtration is conducted, depressurization is conducted to remove solvent, and residues are separated through silica gel column chromatography (petroleum ether/ethyl acetate=4/1) to obtain a yellow oily compound 21 (34.2mg, 67.2%). HPLC: 8.19min, 98.1%; ¹H NMR (400MHz, CDCl₃) δ 7.72 (d, J=8.1Hz, 2H), 7.19 (d, J=8.0Hz, 2H), 6.94 (t, J=7.6Hz, 1H), 6.80 (d, J=7.3Hz, 1H), 6.72-6.64 (m, 2H), 5.02 (s, 2H), 2.87 (s, 3H).

### 22) Synthesis of 4-(4-iodobenzyloxy)-N, N-dimethylaniline (a compound 22)

The compound 16 (162.6mg, 0.5mmol) and paraformaldehyde (150.0mg, 5.0mmol) are dissolved in 20ml of acetic acid, NaBH₃CN (157.0mg, 2.5mmol) is slowly added, and reaction is conducted for 24h at room temperature under a stirring state. 20ml of 1M NaOH solution is added, white precipitate is separated out, suction filtration is conducted, the precipitate is washed with water and methanol recrystallization is conducted to obtain white solid 22 (168.4mg, 95.4%). HPLC: 10.63min, 98.4%; mp 128.2-129.3 °C; ¹H NMR (400MHz, CDCl3) δ 7.69 (d, J=8.2Hz, 2H), 7.17 (d, J=8.0Hz, 2H), 6.88 (d, J=8.9Hz, 2H), 6.82-6.68 (s, 2H), 4.95 (s, 2H), 2.87 (s, 6H).

### 23) Synthesis of 3-(4-iodobenzyloxy)-N, N-dimethylaniline (a compound 23)

Preparation is conducted according to the preparation method of the compound 22 (reactant compound 16 is replaced by the compound 17) to obtain white solid 23 (171.3mg, 97.1%). HPLC: 11.30min, 99.9%; mp 68.8-70.1 °C; 1H NMR (400MHz, CDCl₃) δ 7.71 (d, J = 8.2Hz, 2H), 7.19 (d, J = 8.2Hz, 2H), 7. 15 (t, J = 8.iHz, 1H), 6.43-6.30 (m, 3H), 5.00 (s, 2H), 2.94 (s, 6H). ¹³C NMR (101MHz, CDCl₃) δ 150.92, 145.99, 137.59, 137.41, 129.28, 115.95, 114.77, 93.19, 70.16,41.73.

### 24) Synthesis of 1-iodo-4-(4-methoxybenzyloxy) benzene (a compound 24)

Preparation is conducted according to the preparation method of the compound 1 (reactant 4-nitrophenol is replaced by 4-iodophenol and reactant 4-iodobenzyl bromide is replaced by 4-methoxybenzyl bromide) to obtain white solid 24 (906.3mg, 88.9%). HPLC: 8.55min, 99.7%; mp 130.3-131.5 °C; 1H NMR (400MHz, CDCl₃) δ 7.55 (d, J=9.0Hz, 2H), 7.33 (d, J=8.7Hz, 2H), 6.91 (d, J=8.7Hz, 2H), 6.74 (d, J=8.9Hz, 2H), 4.95 (s, 2H), 3.81 (s, 3H). ¹³C NMR (101MHz, CDCl₃) δ 159.59, 158.71, 138.22, 129.19, 128.55, 117.36, 114.08, 82.95, 69.91, 55.31.

### 25) Synthesis of (4-iodobenzyl) (4-methoxyphenyl) sulfane (a compound 25)

Preparation is conducted according to the preparation method of the compound 1 (reactant 4-nitrophenol is replaced by 4-methoxythiophenol) to obtain white solid 25 (712.4mg, 94.7%). ¹H NMR (400MHz, CDCl₃) δ 7.56 (d, J=8.3Hz, 2H), 7.23 (d, J=8.8Hz, 2H), 6.90 (d, J=8.3Hz, 2H), 6.79 (d, J=8.8Hz, 2H), 3.89 (s, 2H), 3.78 (s, 3H). ¹³C NMR (101MHz, CDCl₃) δ 159.43, 138.01, 137.40, 134.36, 130.81, 125.45, 114.54, 92.31, 55.31,40.77.

### 26) Synthesis of 1-bromo-4-((4-methoxyphenoxy) methyl) benzene (a compound 26)

Preparation is conducted according to the preparation method of the compound 1 (reactant 4-nitrophenol is replaced by 4-methoxyphenol and reactant 4-iodobenzyl bromide is replaced by p-bromobenzyl bromide) to obtain white solid 26 (2.93g, 93.2%). mp 105.3-106.7 °C ; ¹H NMR (400MHz, CDCl₃) δ 7.51 (d, J=8.3Hz, 2H), 7.30 (d, J=8.3Hz, 2H), 6.89 (d, J=9.2Hz, 2H), 6.83 (d, J=9.2Hz, 2H), 4.97 (s, 2H), 3.77 (s, 3H).

### 27) Synthesis of 1-bromo-4-(4-methoxybenzyloxy) benzene (a compound 27)

Preparation is conducted according to the preparation method of the compound 1 (reactant 4-nitrophenol is replaced by 4-bromophenol and reactant 4-iodobenzyl bromide is replaced by 4-methoxybenzyl bromide) to obtain white solid 27 (681.4mg,77.8%). mp 122.1-122.9 °C; ¹H NMR (400MHz, CDCl₃) δ 7.37 (d, J=9.0Hz, 2H), 7.33 (d, J=8.7Hz, 2H), 6.91 (d, J=8.7Hz, 2H), 6.84 (d, J=9.0Hz, 2H), 4.96 (s, 2H), 3.82 (s, 3H).

### 28) Synthesis of 4-(4-bromobenzyloxy)-N, N-dimethylaniline (a compound 28)

Preparation is conducted according to the preparation method of the compound 1 (reactant 4-nitrophenol is replaced by 4-hydroxy-N, N-dimethylaniline and reactant 4-iodobenzyl bromide is replaced by p-bromobenzyl bromide) to obtain white solid 28 (303.5mg, 99.1%). mp 125.8-127.1 °C; ¹H NMR (400MHz, CDCl₃) δ 7.52-7.48 (m, 2H),7.32-7.28 (m, 2H), 6.92-6.85 (m, 2H), 6.76 (s, 2H), 4.96 (s, 2H), 2.88 (s, 6H).

### 29) Synthesis of 1-(benzyloxy)-4-(2-fluoroethoxy) benzene (a compound 29)

4-benzyloxyphenol (2.00g, 10.0mmol) and KOH (0.56g, 10.0mmol) are dissolved in 30ml of anhydrous ethanol, reflux is conducted for 30min by heating at 80 °C, 20ml of 1-bromo-2-fluoroethane (1.52g, 12.0mmol) ethanol solution, continuously reaction is conducted for 1h under a reflux and stirring state, reaction is monitored through TLC till basic completion, depressurization is conducted to remove solvent, 50ml of 1M NaOH solution is added, white precipitate is separated out, suction filtration is conducted, the precipitate is washed with water and methanol recrystallization is conducted to obtain white crystal 29 (2.23g, 90.4%). ¹H NMR (400MHz, CDCl₃) δ 7.46-7.28 (m, 5H), 6.91 (d, J=9.2Hz, 2H), 6.86 (d, J=9.3Hz, 2H), 5.02 (s, 2H), 4.81-4.64 (m, 2H), 4.22-4.10 (m, 2H).

### 30) Synthesis of 4-(2-fluoroethoxy) phenol (a compound 30)

The compound 29 (2.08g, 8.44mmol) is dissolved in 10ml of anhydrous methanol, palladium-carbon catalyst (89.4mg, 0.84mmol) is added, reaction is conducted for 4h at 50 °C under a stirring state under the condition of 1 atm H₂, reaction is monitored through TLC till completion, suction filtration is conducted to remove the palladium-carbon catalyst, and depressurization is conducted to remove solvent to obtain white solid 30 (1.32g, 57.4%). ¹H NMR (400MHz, CDCl₃) δ 6.83 (d, J=8.9Hz, 2H), 6.77 (d, J=9.0Hz, 2H), 4.80-4.64 (m, 2H), 4.42 (s, 1H), 4.23-4.09 (m, 2H).

### 31) Synthesis of 1-(2-fluoroethoxy)-4-((4-iodobenzyl) oxy) benzene (a compound 31a)

The compound 30 (468.5mg, 3.0mmol) and 4-iodobenzyl bromide (890.8mg, 3.0mmol) are dissolved in 5ml of anhydrous DMF, and K₂CO₃ (414.6mg, 3.0mmol) is added. Reaction is conducted for 0.5h at 90 °C under a reflux and stirring state, reaction is monitored through TLC till completion, depressurization is conducted to remove solvent, 50ml of deionized water is added, white precipitate is separated out, suction filtration is conducted, the precipitate is washed with water, and methanol recrystallization is conducted to obtain white solid 31a (986.8mg, 88.4%). ¹H NMR (400MHz, CDCl₃) δ 7.70 (d, J=8.3Hz, 2H), 7.16 (d, J =8.2Hz, 2H), 6.90-6.84 (m, 4H), 4.96 (s, 2H), 4.79-4.65 (m, 2H), 4.21-4.11 (m, 2H). ¹³C NMR (101MHz, CDCl₃) δ 153.11, 152.93, 137.62, 136.94, 129.24, 115.89, 115.79, 93.35, 82.86, 81.17, 69.96, 67.97, 67.76.

### 32) Synthesis of 1-bromo-4-((4-(2-fluoroethoxy) phenoxy) methyl) benzene (a compound 31b)

Preparation is conducted according to the preparation method of the compound 31a (reactant 4-iodobenzyl bromide is replaced by p-bromobenzyl bromide) to obtain white solid 31b (1.55g, 95.2%). ¹H NMR (400MHz, CDCl₃) δ 7.50 (d, J=8.4Hz, 2H), 7.29 (d, J=8. 4Hz, 2 H), 6.90-6.84 (m, 4H), 4.97(s, 2H), 4.80-4.65 (m, 2H), 4.21-4.11(m, 2H).

### 33) Synthesis of 4-(2-fluoroethoxy) benzaldehyde (a compound 32)

P-hydroxybenzaldehyde (2.44g, 20mmol) and 1-bromo-2-fluoroethane (2.54g, 20mmol) are dissolved in 5ml of anhydrous DMF, and K₂CO₃ (5.53g, 40mmol) is added. Reaction is conducted for 2h at 90 °C under a reflux and stirring state, reaction is monitored through TLC till completion, depressurization is conducted to remove solvent, 50ml of deionized water is added, extraction is conducted by using ethyl acetate (3x10ml), drying is conducted by using anhydrous MgSO₄, suction filtration is conducted, and depressurization is conducted to remove solvent to obtain yellow oily liquid 32 (2.95g, 87.8%). ¹H NMR (400MHz, CDCl₃) δ 9.90 (s, 1H), 7.86 (d, J=8.7Hz, 2H), 7.04 (d, J=8.6Hz, 2H), 4.88-4.71 (m, 2H), 4.37-4.24 (m, 2H).

### 34) Synthesis of (4-(2-fluoroethoxy) phenyl) methanol (a compound 33)

The compound 32 (2.95g, 17.6mmol) is dissolved in 10ml of anhydrous methanol, NaBH₄ (1.33g, 35.2mmol) is slowly added at 0 °C under a stirring state, continuously reaction is conducted for 0.5h, reaction is monitored through TLC till completion, 10ml of deionized water is added to quench the reaction, depressurization is conducted to remove methanol, a proper amount of 1M HCl is added to neutralize to pH=7, extraction is conducted by using CH₂Cl₂ (3x10ml), organic phases are combined, drying is conducted by using anhydrous MgSO₄, suction filtration is conducted, and depressurization is conducted to remove solvent to obtain yellow oily liquid 33 (2.73g, 91.1%). ¹H NMR(400MHz, CDCl₃) δ 7.31 (d, J=8.5Hz, 2H), 6.93 (d, J=8.5Hz, 2H), 4.84-4.68 (m, 2H), 4.63 (s, 2H), 4.27-4.17(m, 2H).

### 35) Synthesis of 1-(bromomethyl)-4-(2-fluoroethoxy) benzene (a compound 34)

The compound 33 (2.73g, 16.0mmol) is dissolved in 25ml of anhydrous CH₂Cl₂, 25ml of PBr₃(4.33g, 16.0mmol) CH₂Cl₂ solution is slowly added at room temperature under a stirring state, continuously reaction is conducted for 0.5h, reaction is monitored through TLC till completion, 20ml of deionized water is added to quench the reaction, then 1g of NaHCO₃ is added, stirring is continuously conducted for 0.5h, extraction is conducted by using CH₂Cl₂ (3x10ml), organic phases are combined, drying is conducted by using anhydrous MgSO₄, suction filtration is conducted, and depressurization is conducted to remove solvent to obtain colorless oily liquid 34 (3.54g, 95.0%). ¹H NMR (400MHz, CDCl₃) δ 7.33 (d, J=8.6Hz, 2H), 6.89 (d, J = 8.6Hz, 2H), 4.84-4.68 (m, 2H), 4.50 (s, 2H), 4.27-4.16 (m, 2H).

### 36) Synthesis of 1-(2-fluoroethoxy)-4-((4-iodophenoxy) methyl) benzene (a compound 35a)

Preparation is conducted according to the preparation method of the compound 31a (reactant 4-iodobenzyl bromide is replaced by the compound 34 and reactant compound 30 is replaced by 4-iodophenol) to obtain white solid 35a (623.4mg, 94.1%). ¹H NMR (400MHz, CDCl₃) δ 7.55 (d, J= 8.9Hz, 2H), 7.34 (d, J=8.6Hz, 2H), 6.94 (d, J=8.6Hz, 2H), 6.74 (d, J=8.9Hz, 2H), 4.95 (s, 2H), 4.83-4.68(m, 2H), 4.27-4.16 (m, 2H). ¹³C NMR (101MHz, CDCl₃) δ 158.65, 158.39, 138.23, 129.24, 129.21, 117.34, 114.81, 83.00, 82.72, 81.03, 69.80, 67.31, 67.10.

### 37) Synthesis of 1-bromo-4-((4-(2-fluoroethoxy) benzyl) oxy) benzene (a compound 35b)

Preparation is conducted according to the preparation method of the compound 31a (reactant 4-iodobenzyl bromide is replaced by the compound 34 and reactant compound 30 is replaced by 4-bromophenol) to obtain white solid 35b (1.03g, 76.1%). ¹H NMR (400MHz, CDCl₃) δ 7.35 (7.39-7.31, 4H), 6.94 (d, J=8.0Hz, 2H), 6.84 (d, J=8.2Hz, 2H), 4.96 (s, 2H), 4.76 (d, J=47.3Hz, 2H), 4.22 (d, J=27.8Hz, 2H).

### 38) Synthesis of tributyl (4-((4-methoxyphenoxy) methyl) phenyl) stannane (a compound 36)

The compound 26 (146.6mg, 0.5mmol), hexabutyldistannane (580.1mg, 1.0mmol) and Tetrakis (triphenylphosphine) palladium (57.8mg, 0.05mmol) are dissolved in 10ml of methylbenzene (containing 1ml of triethylamine), and reaction is conducted over a night under a reflux and stirring state. Depressurization is conducted to remove solvent, and residues are separated through silica gel column chromatography (petroleum ether/ethyl acetate=15/1) to obtain a colorless oily compound 36 (89.5mg, 35.6%). ¹HNMR (400MHz, CDCl₃) δ 7.49 (d, J=7.9Hz, 2H), 7.39 (d, J=7.8Hz, 2H), 6.93 (d, J=9.1Hz, 2H), 6.85 (d, J=9.1Hz, 2H), 5.00 (s, 2H), 3.78 (s, 3H), 1.59-1.51 (m, 6H), 1.39-1.29 (m, 6H), 1.15-0.97 (m, 6H), 0.90 (t, J=7.3Hz, 9H).

### 39) Synthesis of tributyl (4-(4-methoxybenzyloxy) phenyl) stannane (a compound 37)

Preparation is conducted according to the preparation method of the compound 36 (reactant compound 26 is replaced by the compound 27) to obtain a colorless oily compound 37(75.0mg, 29.8%). ¹H NMR (400MHz, CDCl₃) δ 7.40-7.32 (m, 3H), 7.31-7.27 (m, 1H), 6.99-6.95 (m, 2H), 6.91 (d, J=8.4Hz, 2H), 4.98 (s, 2H), 3.82 (s, 3H), 1.69-1.60 (m, 6H), 1.41-1.30 (m, 12H), 0.92 (t, J=7.3Hz, 9H).

### 40) Synthesis of N, N-dimethyl-4-(4-(tributylstannyl) benzyloxy) aniline (a compound 38)

Preparation is conducted according to the preparation method of the compound 36 (reactant compound 26 is replaced by the compound 28) to obtain a colorless oily compound 38 (56.2mg, 21.8%). ¹H NMR (400MHz, CDCl₃) δ 7.47 (d, J=7.7Hz, 2H), 7.39 (d, J=7.6Hz, 2H), 6.92 (d, J=9.0Hz, 2H), 6.76 (d, J=8.7Hz, 2H), 4.98 (s, 2H), 2.87 (s, 6H), 1.58- 1.49 (m, 6H), 1.38-1.28 (m, 12H), 0.89 (t, J=7.4Hz, 9H).

### 41) Synthesis of tributyl (4-((4-(2-fluoroethoxy) phenoxy) methyl) phenyl) stannane (a compound 39)

Preparation is conducted according to the preparation method of the compound 36 (reactant compound 26 is replaced by the compound 31b) to obtain a colorless oily compound 39 (325.6mg, 30.4%). ¹H NMR (400MHz, CDCl₃) δ 7.54-7.41 (m, 2H), 7.37 (d, J=7.8Hz, 2H), 6.92 (d, J=9.3Hz, 2H), 6.87 (d, J=9.3Hz, 2H), 4.99 (s, 2H) ,4.80-4.65 (m, 2H), 4.22-4.11 (m, 2H), 1.58- 1.50 (m, 6H), 1.39-1.27 (m, 6H), 1.14-0.96 (m, 6H), 0.88 (t, J=7.3Hz, 9H).

### 42) Synthesis of tributyl (4-((4-(2-fluoroethoxy) benzyl) oxy) phenyl) stannane (a compound 40)

Preparation is conducted according to the preparation method of the compound 36 (reactant compound 26 is replaced by the compound 35b) to obtain a colorless oily compound 40 (134.6mg, 25.1%). ¹H NMR (400MHz, CDCl₃) δ 7.37 (d, J=8.7Hz, 2H), 7.32-7.26 (m, 2H), 6.99-6.93 (m, 4H), 5.00 (s, 2H), 4.83-4.68 (m, 2H), 4.28-4.17 (m, 2H), 1.58-1.50 (m, 6H), 1.39-1.27 (m, 6H), 1.14-0.96 (m, 6H), 0.88(t, J=7.3Hz, 9H).

### Embodiment 2: synthesis of fluoro compounds

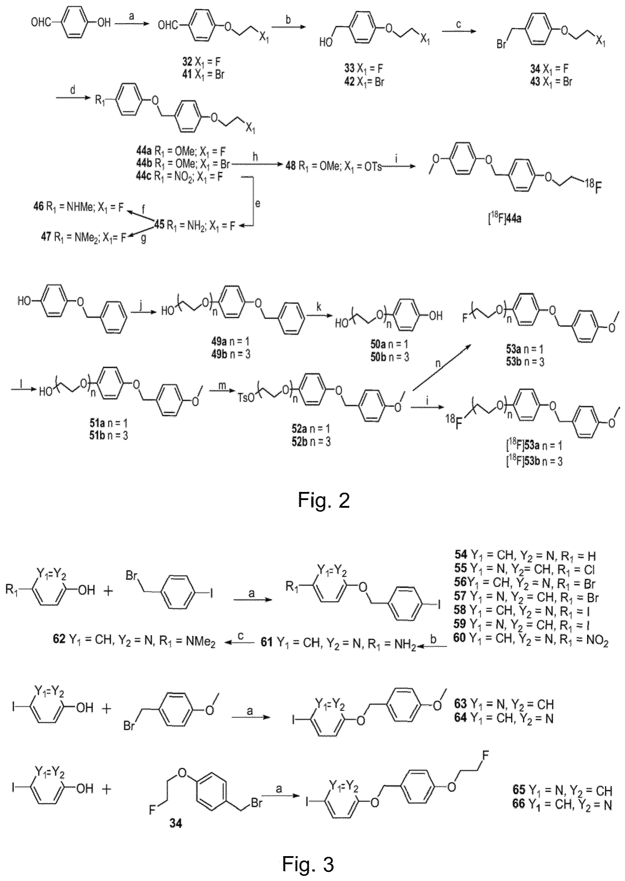

A synthesis reaction route is shown in Fig.2. Serial numbers of compounds in the embodiment 2 are consistent with serial numbers in the reaction route in Fig.2.

In the synthesis route shown in Fig.2, reagents and conditions are as follows:
(a) 1-bromo-2-fluoroethane or 1, 2-dibromoethane, K₂CO₃, DMF, 90 °C; (b) NaBH₄, MeOH, 0 °C; (c) PBr₃, CH₂Cl₂, r. t.; (d) 4-methoxyphenol or 4-nitrophenol, K₂CO₃, DMF, 90 °C; (e) SnCl₂•2H₂O, EtOH, HCl,reflux; (f) 1: NaOMe, (CH₂O)ₙ, MeOH, reflux; 2: NaBH₄, reflux; (g) (CH₂O)ₙ, NaBH₃CN, HAc, r.t.; (h) AgOTs, MeCN, reflux; (i) ¹⁸F⁻, K₂CO₃, Kryptofix-2.2.2 (i.e., K₂₂₂, aminopolyether), acetonitrile, 100 °C; (j) 2-chloroethanol or 2-(2-(2-chloroethoxy) ethoxy) ethanol), KOH, EtOH, reflux; (k) 10%Pd/C,1atm H₂, 50 °C;(1)1-(chloromethyl)-4- methoxybenzene (i.e., 4-methoxybenzyl chloride), K₂CO₃, DMF, 90 °C; (m) TsCl, CH₂Cl₂, Et₃N, 0 °C-rt; (n)TBAF (1M in THF), THF, reflux.

### 1) Synthesis of 4-(2-bromoethoxy) benzaldehyde (a compound 41)

P-hydroxybenzaldehyde (2.44g, 20mmol) and 1, 2-dibromoethane (7.51g, 40mmol) are dissolved in 5ml of anhydrous DMF, and K₂CO₃ (5.53g, 40mmol) is added. Reaction is conducted for 2h at 90 °C under a reflux and stirring state, reaction is monitored through TLC till completion, depressurization is conducted to remove solvent, 50ml of deionized water is added, extraction is conducted by using CH₂Cl₂ (3x10ml), organic phases are combined, drying is conducted by using anhydrous MgSO₄, suction filtration is conducted, depressurization is conducted to remove solvent, and residues are separated through silica gel column chromatography (petroleum ether/ethyl acetate=4/1) to obtain white solid 41 (1.32g, 28.9%). ¹H NMR (400MHz, CDCl₃) δ 9.90 (s, 1H), 7.85 (d, J=8.8Hz, 2H), 7.02 (d, J= 8.7Hz, 2H), 4.38 (t, J=6.2Hz, 2H), 3.67 (t, J=6.2Hz, 2H).

### 2) Synthesis of (4-(2-bromoethoxy) phenyl) methanol (a compound 42)

Preparation is conducted according to the preparation method of the compound 33 (reactant compound 32 is replaced by the compound 41) to obtainwhite solid 42 (1.14g, 95.8%). ¹H NMR (400MHz,CDCl₃) δ 7.30 (d, J=8.5Hz, 2H), 6.91(d, J=8.6Hz, 2H), 4.63 (s, 2H), 4.30 (t, J=6.3Hz, 2H), 3.64 (t, J=6.3Hz, 2H).

### 3) Synthesis of 1-(2-bromoethoxy)-4-(bromomethyl) benzene (a compound 43)

Preparation is conducted according to the preparation method of the compound 34 (reactant compound 33 is replaced by the compound 42) to obtainwhite solid 43 (1.29g, 99.0%).¹H NMR (400MHz, CDCl₃) δ 7.33 (d, J=8.7Hz, 2H), 6.87 (d, J=8.7Hz, 2H), 4.49 (s, 2H), 4.29 (t, J=6.3Hz, 2H), 3.63 (t, J=6.3Hz, 2H).

### 4) Synthesis of 1-(2-fluoroethoxy)-4-((4-methoxyphenoxy) methyl) benzene (a compound 44a)

The compound 34 (411.3mg, 1.76mmol) and 4-methoxyphenol (218.5mg, 1.76mmol) are dissolved in 5ml of anhydrous DMF, and K₂CO₃ (243.2mg, 1.76mmol) is added. Reaction is conducted for 0.5h at 90 °C under a reflux and stirring state, reaction is monitored through TLC till completion, depressurization is conducted to remove solvent, 50ml of deionized water is added, white precipitate is separated out, suction filtration is conducted, the precipitate is washed with water and methanol recrystallization is conducted to obtain white solid 44a (136.5mg, 28.1%). ¹H NMR (400MHz, CDCl₃) δ 7.35 (d, J=8.5Hz, 2H), 6.97-6.87 (m, 4H), 6.83 (d, J= 9.1Hz, 2H), 4.94 (s, 2H), 4.84-4.67 (m, 2H), 4.28-4.15 (m, 2H), 3.77 (s, 3H). ¹³C NMR (101MHz, CDCl₃) δ 158.24, 154.01, 152.99, 130.09, 129.20, 115.95, 114.74, 114.67, 82.74, 81.04, 70.45, 67.31, 67.10, 55.71.

### 5) Synthesis of 1-(2-bromoethoxy)-4-((4-methoxyphenoxy) methyl) benzene (a compound 44b)

Preparation is conducted according to the preparation method of the compound 44a (reactant compound 34 is replaced by the compound 43) to obtain white solid 44b (242.8mg, 68.1%). ¹H NMR (400MHz, CDCl₃) δ 7.35 (d, J=8.6Hz, 2H), 6.94-6.87 (m, 4H), 6.83 (d, J=9.2Hz, 2H), 4.94 (s, 2H), 4.30 (t, J=6.3Hz, 2H), 3.77 (s, 3H), 3.63 (t, J=6.3Hz, 2H).

### 6) Synthesis of 1-(2-fluoroethoxy)-4-((4-nitrophenoxy) methyl) benzene (a compound 44c)

Preparation is conducted according to the preparation method of the compound 44a (reactant 4-methoxyphenol is replaced by 4-nitrophenol) to obtain white solid 44c (2.35g, 78.6%). ¹H NMR (400MHz, CDCl₃) δ 8.20 (d, J=9.2Hz, 2H), 7.36 (d, J=8.5Hz, 2H), 7.02 (d, J=9.2Hz, 2H), 6.96 (d, J=8.5Hz, 2H), 5.09 (s, 2H), 4.86-4.67 (m, 2H), 4.31-4.17 (m, 2H).

### 7) Synthesis of 4-(4-(2-fluoroethoxy) benzyloxy) aniline (a compound 45)

The compound 44c (1.94g, 6.67mmol) and SnCl₂•2H₂O (3.01g, 13.34mmol) are dissolved in 25ml of ethanol, 2ml of concentrated hydrochloric acid is dripped, reaction is conducted for 2h at 80 °C under a reflux and stirring state, cooling to room temperature is conducted, 30ml of 1M NaOH water solution is added to neutralize the hydrochloric acid and precipitate SnCl₂, extraction is conducted by using ethyl acetate (3x10ml), organic phases are combined, drying is conducted by using anhydrous MgSO₄, suction filtration is conducted, and depressurization is conducted to remove solvent to obtain white solid 45 (1.26g, 72.5%). ¹H NMR (400MHz, CDCl₃) δ 7.34 (d, J=8.4Hz, 2H), 6.92 (d, J=8.5Hz, 2H), 6.80 (d, J=8.6Hz, 2H), 6.63 (d, J=8.6Hz, 2H), 4.91 (s, 2H), 4.84-4.67 (m, 2H), 4.27-4.15 (m, 2H), 3.42 (s, 2H).

### 8) Synthesis of 4-(4-(2-fluoroethoxy) benzyloxy)-N-methylaniline (a compound 46)

The compound 45 (522.6mg, 2.0mmol) and paraformaldehyde(240.0mg, 8.0mmol) are dissolved in 30ml of anhydrous methanol, 5ml of NaOCH₃ (216.1mg, 4.0mmol) methanol solution is added drop by drop, and reaction is conducted for 2h under a reflux and stirring state. After cooling to room temperature is conducted, NaBH₄ (302.4mg, 8.0mmol) is slowly added and continuously reaction is conducted for 2h under a reflex and stirring state. Depressurization is conducted to remove solvent, 50ml of 1M NaOH solution is added, white precipitate is separated out, suction filtration is conducted, the precipitate is washed with water, and methanol recrystallization is conducted to obtain light yellow crystal 46 (423.6mg, 76.9%). ¹H NMR (400MHz, CDCl₃) δ 7.34 (d, J=4.8Hz, 2H), 6.92 (d, J=4.8Hz, 2H), 6.85 (d, J=5.0Hz, 2H), 6.57 (d, J=4.9Hz, 2H), 4.92 (s, 2H), 4.75 (d, J=47.4Hz, 2H), 4.22 (d, J=27.6Hz, 2H), 3.49 (s, 1H), 2.80 (s, 3H).

### 9) Synthesis of 4-(4-(2-fluoroethoxy) benzyloxy)-N, N-dimethylaniline (a compound 47)

The compound 45 (261.3mg, 1.0mmol) and paraformaldehyde (300.0mg, 10.0mmol) are dissolved in 20ml of acetic acid, NaBH₃CN (314.0mg, 5.0mmol) is slowly added and reaction is conducted for 24h at room temperature under a stirring state. 20ml of 1M NaOH solution is added, white precipitate is separated out, suction filtration is conducted, the precipitate is washed with water, and methanol recrystallization is conducted to obtain light yellow crystal 47 (213.9mg, 75.8%). ¹H NMR (400MHz, CDCl₃) δ 7.35 (d, J=8.0Hz, 2H), 6.96-6.87 (m, 4H), 6.77 (s, 2H), 4.94 (s, 2H), 4.75 (d, J=47.7Hz, 2H), 4.22 (d, J=27.7Hz, 2H), 2.88 (s, 6H).

### 10) Synthesis of 2-(4-((4-methoxyphenoxy) methyl) phenoxy) ethyl-4-methylbenzenesulfonate (a compound 48)

The compound 44b (137.2mg, 0.41mmol) and silver p-methylbenzene sulfonate (227.1mg, 0.82mg) are dissolved in 20ml of acetonitrile, reaction is conducted for 12h at 90 °C under a reflux and stirring state, reaction is monitored through TLC till basic completion, depressurization is conducted to remove solvent, and residues are separated through silica gel column chromatography (petroleum ether/ethyl acetate=4/1) to obtain white solid 48 (174.4mg, 60.6%). ¹H NMR (400MHz, CDCl₃) δ 7.82 (d, J=8.3Hz, 2H), 7.34 (d, J=8.1Hz, 2H), 7.31 (d, J=8.6Hz, 2H),6.89 (d, J=9.2Hz, 2H), 6.82 (d, J=9.2Hz, 2H), 6.78 (d, J=8.6Hz, 2H), 4.92 (s, 2H) ,4.39-4.35(m, 2H), 4.17-4.13 (m, 2H), 3.77 (s, 3H), 2.45 (s, 3H). ¹³C NMR (101MHz, CDCl₃) δ 157.80, 154.01, 152.94, 144.94, 132.96, 130.21, 129.86, 129.12,127.99, 115.92 (overlapped), 114.66, 70.39, 68.09, 65.52, 55.72, 21.62.

### 11) Synthesis of 2-(4-(benzyloxy) phenoxy) ethanol (a compound 49a)

The 4-benzyloxyphenol (4.00g, 20.0mmol) and KOH(1.12g, 20.0mmol) are dissolved in 30ml of anhydrous ethanol, reflux is conducted for 30min by heating at 80 °C, 20ml of 2-chloroethanol (2.01g, 25mmol) ethanol solution, continuously reaction is conducted for 1h under a reflux and stirring state, reaction is monitored through TLC till basic completion, depressurization is conducted to remove solvent, 50ml of 1M NaOH solution is added, white precipitate is separated out, suction filtration is conducted, the precipitate is washed with water and methanol recrystallization is conducted to obtain white crystal 49a (2.35g, 48.1%). ¹H NMR (400MHz, CDCl₃) δ 7.46-7.30 (m, 5H), 6.91 (d, J=9.1Hz, 2H), 6.85 (d, J=9.0Hz, 2H), 5.02 (s, 2H), 4.08-3.99 (m, 2H), 3.97-3.87 (m, 2H), 1.95 (s, 1H).

### 12) Synthesis of 2-(2-(2-(4-(benzyloxy) phenoxy) ethoxy) ethoxy) ethanol (a compound 49b)

Preparation is conducted according to the preparation method of the compound 49a (reactant 2-chloroethanol is replaced by 2-(2-(2-chloroethoxy) ethoxy) ethanol) to obtain white solid 49b (2.55g, 38.3%). ¹H NMR (400MHz, CDCl₃) δ 7.46-7.29 (m, 5H), 6.93-6.81 (m, 4H), 5.01 (s, 2H), 4.12-4.02 (m, 2H), 3.86-3.79 (m, 2H), 3.75-3.67 (m, 6H), 3.64-3.59 (m,2H), 2.16 (s, 1H).

### 13) Synthesis of 4-(2-hydroxyethoxy) phenol (a compound 50a)

The compound 49a (2.15g, 8.79mmol) is dissolved in 10ml of anhydrous methanol, palladium-carbon catalyst (93.6mg, 0.88mmol) is added, reaction is conducted for 4h at 50 °C under a stirring state under the condition of 1 atm H₂, reaction is monitored through TLC till completion, suction filtration is conducted to remove the palladium-carbon catalyst, and depressurization is conducted to remove solvent to obtain white solid 50a (1.35g, 100%). ¹HNMR (400MHz, DMSO) δ 8.86 (s, 1H), 6.74 (d, J=8.6Hz, 2H), 6.66 (d, J=8.8Hz, 2H), 4.78 (t, J=5.2Hz, 1H),3.90-3.80 (m,2H), 3.70-3.61 (m, 2H).

### 14) Synthesis of 4-(2-(2-(2-hydroxyethoxy) ethoxy) ethoxy) phenol (a compound 50b)

Preparation is conducted according to the preparation method of the compound 50a (reactant compound 49a is replaced by the compound 49b) to obtain a colorless transparent oily compound 50b (1.78g, 98.6%). ¹H NMR(400MHz, CDCl₃) δ 7.26 (s,1H),6.81-6.67 (m, 4H),4.08-4.01 (m, 2H),3.86-3.79 (m, 2H),3.77-3.68 (m, 6H), 3.66-3.59(m,2H).

### 15) Synthesis of 2-(4-(4-methoxybenzyloxy) phenoxy) ethanol (a compound 51a)

The compound 50a (235.3mg, 1.53mmol) and 4-methoxybenzyl chloride (239.6mg, 1.53mmol) are dissolved in 5ml of anhydrous DMF, andK₂CO₃ ((211.6mg, 1.53mmol) is added. Reaction is conducted for 0.5h at 90 °C under a reflux and stirring state, reaction is monitored through TLC till completion, depressurization is conducted to remove solvent, deionized water is added, white precipitate is separated out, suction filtration is conducted, the precipitate is washed with water, and methanol recrystallization is conducted to obtain white solid 51a (282.8mg, 67.4%). ¹H NMR (400MHz, CDCl₃) δ 7.34 (d, J=8.6Hz, 2H), 6.94-6.82 (m, 6H), 4.94 (s, 2H), 4.06-4.01 (m, 2H), 3.96-3.90 (m, 2H), 3.81 (s, 3H).

### 16) Synthesis of 2-(2-(2-(4-(4-methoxybenzyloxy) phenoxy) ethoxy) ethoxy) ethanol (a compound 51b)

Preparation is conducted according to the preparation method of the compound 51a (reactant compound 50a is replaced by the compound 50b) to obtainwhite solid 51b (369.7mg, 66.9%). ¹H NMR (400MHz, CDCl₃) δ 7.34 (d, J=8.0Hz, 2H), 6.94-6.82 (m, 2H), 4.93 (s, 2H), 4.12-4.05 (m, 2H), 3.88-3.83 (m, 2H), 3.81 (s, 3H), 3.76-3.68 (m, 6H), 3.65-3.58 (m, 2H), 2.08 (s, 1H).

### 17) Synthesis of 2-(4-(4-methoxybenzyloxy) phenoxy) ethyl-4-methylbenzenesulfonate (a compound 52a)

The compound 51a (137.2mg, 0.5mmol) is dissolved in 10ml of CH₂Cl₂, 10ml of triethylamine is added, p-toluensulfonyl chloride (143.0mg, 0.75mmol) is slowly added under an ice-bath stirring state, continuously reaction is conducted for 4h under a stirring state, reaction is monitored through TLC till completion, depressurization is conducted to remove solvent, 50ml of deionized water is added, extraction is conducted by using CH₂Cl₂ (3x10ml), drying is conducted by using anhydrous MgSO₄, suction filtration is conducted, depressurization is conducted to remove solvent, and residues are separated through silica gel column chromatography (petroleum ether/ethyl acetate=4/1) to obtain white solid 52a (214.3mg, 77.2%). ¹H NMR (400MHz, CDCl₃) δ 7.81 (d, J=8.1Hz, 2H),7.33 (d, J=8.1Hz, 4H), 6.91 (d, J=8.4Hz, 2H), 6.85 (d, J=8.7Hz, 2H),6.72 (d, J=8.9Hz, 2H), 4.92 (s, 2H), 4.37-4.31 (m, 2H), 4.12-4.07 (m, 2H), 3.81 (s, 3H). ¹³C NMR (101MHz, CDCl₃) δ 159.46, 153.57, 152.32, 144.89, 133.01, 129.84, 129.18, 128.00, 115.88, 115.76, 114.00, 70.45, 68.27, 66.26, 55.29, 21.62.

### 18) Synthesis of 2-(2-(2-(4-(4_methoxybenzyloxy)phenoxy)ethoxy)ethoxy) ethyl-4-methylbenzenesulfonate (a compound 52b)

Preparation is conducted according to the preparation method of the compound 52a (reactant compound 51a is replaced by the compound 51b) to obtain colorless transparent oily liquid 52b (225.4mg, 83.9%). ¹H NMR (400MHz, CDCl₃) δ 7.79 (d, J=7.9Hz, 2H), 7.37-7.30 (m, 4H), 6. 93-6.82 (m, 6H), 4.93 (s, 2H), 4.1 9-4.13 (m, 2H), 4.08-4.02 (m, 2H), 3.83-3.76 (m, 5H), 3.71-3.58 (m, 6H), 2.43 (s, 3H). ¹³C NMR (101MHz, CDCl₃) δ 159.44, 153.25, 153.12, 144.76, 133.17, 129.81, 129.36, 129.18, 127.96, 115.91, 115.66, 113.99, 70.81, 70.75, 70.52, 69.93, 69.25, 68.74, 68.13, 55.29, 21.59.

### 19) Synthesis of 1-(2-fluoroethoxy)-4-(4_methoxybenzyloxy) benzene (a compound 53a)

The compound 52a (128.6mg, 0.3mmol) is dissolved in 5ml of anhydrous THF, 0.6ml of tetrabutylammonium fluoride tetrahydrofuran solution (1M) is added. Reaction is conducted for 2h at 80 °C under a reflux and stirring state, reaction is monitored through TLC till completion, depressurization is conducted to remove solvent, and residues are separated through silica gel column chromatography (petroleum ether/ethyl acetate=2/1) to obtain white solid 53a (70.7mg, 85.3%). ¹H NMR (400MHz, CDCl₃) δ 7.34 (d, J=8.2Hz, 2H), 6.94-6.84 (m, 6H), 4.94 (s, 2H), 4.81-4.64 (m, 2H), 4.23-4.11 (m, 2H), 3.81 (s, 3H). ¹³C NMR (101MHz, CDCl₃) δ 159.48, 153.56, 152.80, 129.31, 129.19, 115.99, 115.82, 114.02, 82.90, 81.20, 70.52, 68.06, 67.85, 55.30.

### 20) Synthesis of 1-(2-(2-(2-fluoroethoxy) ethoxy) ethoxy)-4-(4-methoxybenzyloxy) benzene (a compound 53b)

Preparation is conducted according to the preparation method of the compound 53a (reactant compound 52a is replaced by the compound 52b) to obtain colorless transparent oily liquid 53b (87.5mg, 73.7%). ¹H NMR (400MHz, CDCl₃) δ 7.34 (d, J=8.2Hz, 2H), 6.94-6.82 (m, 4H), 4.93 (s, 2H), 4.56 (d, J=47.8Hz, 2H), 4.09 (s, 2H), 3.85-3.70 (m, 11H). ¹³C NMR (101MHz, CDCl₃) δ 159.42, 153.21, 153.13, 129.34, 129.18, 115.87, 115.64, 113.97, 83.97, 82.29, 70.85, 70.84, 70.54, 70.49, 70.34, 69.93, 68.12, 55.28.

### Embodiment 3: synthesis of pyridine derivatives

A synthesis reaction route is shown in Fig.3. Serial numbers of compounds in the embodiment 3 are consistent with serial numbers in the reaction route in Fig.3.

In the synthesis route shown in Fig.3, reagents and conditions are as follows: (a) K₂CO₃, DMF, 90 °C; (b) SnCl₂•2H₂O, EtOH, HCl, reflux; (c) CH₃I, K₂CO₃, r.t.

### 1) Synthesis of 2-((4-iodobenzyl) oxy) pyridine (a compound 54)

Preparation is conducted according to the preparation method of the compound 1 (reactant 4-nitrophenol is replaced by 2-hydroxypyridine) to obtain white solid 54 (187.5mg, 60.3%). ¹H NMR (400MHz, CDCl₃) δ 7.67 (d, J=8.2Hz, 2H), 7.34 (ddd, J=8.8, 6.7, 1.8Hz, 1H), 7.26-7.24 (m, 1H), 7.06 (d, J=8.2Hz, 3H), 6.66 (d, J=9.1Hz, 1H), 6.19 (t,J=6.7Hz, 1H), 5.09 (s, 2H).

### 2) Synthesis of 2-chloro-5-((4-iodobenzyl) oxy) pyridine (a compound 55)

Preparation is conducted according to the preparation method of the compound 1 (reactant 4-nitrophenol is replaced by 2-chloro-5-hydroxypyridine) to obtain white solid 55 (635.7mg, 92.0%). ¹H NMR (400MHz, CDCl₃) δ 8.11 (s, 1H), 7.73 (d, J=8.2Hz, 2H), 7.24-7.21 (m, 2H), 7.16 (d, J=8.1Hz, 2H), 5.04 (s, 2H).

### 3) Synthesis of 5-bromo-2-((4-iodobenzyl) oxy) pyridine (a compound 56)

Preparation is conducted according to the preparation method of the compound 1 (reactant 4-nitrophenol is replaced by 5-bromo-2-hydroxypyridine) to obtain white solid 56 (346.5mg, 88.8%). 1H NMR (400MHz, CDCl₃) δ 7.69 (d, J=8.3Hz, 2H), 7.36-7.32 (m, 2H), 7.06 (d, J=8.2Hz, 2H), 6.54 (d, J=10.5Hz, 1H), 5.03 (s, 2H).

### 4) Synthesis of 2-bromo-5-((4-iodobenzyl) oxy) pyridine (a compound 57)

Preparation is conducted according to the preparation method of the compound 1 (reactant 4-nitrophenol is replaced by 2-bromo-5-hydroxypyridine) to obtain white solid 57 (361.7mg, 92.7%). ¹H NMR (400MHz, CDCl₃) δ 8.11 (d, J=3.1Hz, 1H), 7.73 (d, J=8.2Hz, 2H), 7.37 (d, J=8.7Hz, 1H), 7.17-7.11 (m, 3H), 5.03 (s, 2H).

### 5) Synthesis of 5-iodo-2-((4-iodobenzyl) oxy) pyridine (a compound 58)

Preparation is conducted according to the preparation method of the compound 1 (reactant 4-nitrophenol is replaced by 2-hydroxy-5-iodopyridine) to obtain white solid58 (321.5mg, 73.6%). ¹H NMR (400MHz, CDCl3) δ 7.69 (d, J=8.3Hz, 2H), 7.45 (d, J=2.2Hz, 1H), 7.42 (dd, J=9.5, 2.4Hz, 1H), 7.05 (d, J=8.2Hz, 2H), 6.44 (d, J=9.5Hz, 1H), 5.02 (s, 2H).

### 6) Synthesis of 2-iodo-5-((4-iodobenzyl) oxy) pyridine (a compound 59)

Preparation is conducted according to the preparation method of the compound 1 (reactant 4-nitrophenol is replaced by 2-iodo-5-hydroxypyridine) to obtain white solid59 (289.8mg, 48.8%). ¹H NMR (400MHz, CDCl₃) δ 8.06-8.02 (m, 1H), 7.75 (d, J=8.2Hz, 2H), 7.24 (d, J=8.4Hz, 2H), 7.21-7.15 (m, 1H), 7.03-6.98 (m, 1H), 5.12 (s, 2H).

### 7) Synthesis of 2-((4-iodobenzyl) oxy)-5-nitropyridine (a compound 60)

Preparation is conducted according to the preparation method of the compound 1 (reactant 4-nitrophenol is replaced by 2-hydroxy-5-nitropyridine) to obtain yellow solid 60 (343.0mg, 96.3%). ¹H NMR (400MHz, CDCl₃) δ 8.58 (d, J=3.0Hz, 1H), 8.08(dd, J=10.1,3.0Hz, 1H), 7.73 (d, J=8.2Hz, 2H), 7.11 (d, J=8.2Hz, 2H), 6.60 (d, J=10.1Hz, 1H), 5.12 (s, 2H).

### 8) Synthesis of 6-((4-iodobenzyl) oxy) pyridin-3-amine (a compound 61)

Preparation is conducted according to the preparation method of the compound 16 (reactant compound 1 is replaced by the compound 60) to obtain blue solid 61 (203.4mg, 82.0%). ¹H NMR (400MHz, CDCl₃) δ 7.66 (d, J=8.2Hz, 2H), 7.06-7.02 (m, 3H), 6.70 (d, J=2.9Hz, 1H), 6.57 (d, J=9.6Hz, 1H), 5.01 (s, 2H).

### 9) Synthesis of 6-((4-iodobenzyl) oxy)-N, N-dimethylpyridin-3-amine (a compound 62)

Preparation is conducted according to the preparation method of the compound 21 (reactant compound 18 is replaced by the compound 61) to obtain blue solid 62 (66.8mg, 35.6%). ¹H NMR (400MHz, CDCl₃) δ 7.67 (d, J=8.3Hz, 3H), 7.34 (d, J=9.7Hz, 1H),7.09 (d, J=8.0Hz, 2H), 6.67 (d, J=9.8Hz, 1H), 5.06 (s, 2H), 2.82 (s, 6H).

### 10) Synthesis of 2-iodo-5-((4-methoxybenzyl) oxy) pyridine (a compound 63)

Preparation is conducted according to the preparation method of the compound 1 (reactant 4-nitrophenol is replaced by 2-iodo-5-hydroxypyridine and reactant 4-iodobenzyl bromide is replaced by 4-methoxybenzyl bromide) to obtain white solid63 (569.1mg, 83.4%). ¹H NMR (400MHz, CDCl₃) δ 8.01 (dd, J=4.6, 1.4Hz, 1H), 7.39 (d, J=8.6Hz, 2H), 7.16 (dd, J=8.1, 4.6Hz, 1H), 7.05-7.01 (m, 1H), 6.93 (d, J=8.7Hz, 2H), 5.11 (s, 2H), 3.82 (s, 3H).

### 11) Synthesis of 5-iodo-2-((4-methoxybenzyl) oxy) pyridine (a compound 64)

Preparation is conducted according to the preparation method of the compound 1 (reactant 4-nitrophenol is replaced by 2-hydroxy-5-iodopyridine and reactant 4-iodobenzyl bromide is replaced by 4-methoxybenzyl bromide) to obtain white solid 64(297.4mg, 87.2%). ¹H NMR (400MHz, CDCl₃) δ 7.46 (d, J=2.4Hz, 1H), 7.40 (dd, J=9.5,2.4Hz, 1H), 7.26 (d, J=8.6Hz, 2H), 6.89 (d, J=8.6Hz, 2H), 6.45 (d, J=9.5Hz, 1H), 5.02 (s, 2H), 3.80 (s, 3H).

### 12) Synthesis of 5-((4-(2-fluoroethoxy) benzyl) oxy)-2-iodopyridine (a compound 65)

Preparation is conducted according to the preparation method of the compound 1 (reactant 4-nitrophenol is replaced by 2-iodo-5-hydroxypyridine and reactant 4-iodobenzyl bromide is replaced by the compound 34) to obtain white solid65(357.4mg, 73.1%). ¹H NMR (400MHz, CDCl₃) δ 8.05 (d, J=4.7Hz, 1H), 7.40 (d, J=8.6Hz, 2H), 7.21 (dd, J=8.0, 4.8Hz, 1H), 7.07 (d, J=8.2Hz, 1H), 6.96 (d, J=8.7Hz, 2H), 5.13 (s, 2H), 4.85-4.68 (m, 2H), 4.29-4.18 (m, 2H).

### 13) Synthesis of 2-((4-(2-fluoroethoxy) benzyl) oxy)-5-iodopyridine (a compound 66)

Preparation is conducted according to the preparation method of the compound 1 (reactant 4-nitrophenol is replaced by 2-hydroxy-5-iodopyridine and reactant 4-iodobenzyl bromide is replaced by the compound 34) to obtain white solid 66 (297.8mg, 79.8%). ¹H NMR (400MHz, CDCl₃) δ 7.47 (s, 1H), 7.43 (d, J=9.5Hz, 1H), 7.29-7.26 (m, 2H), 6.92 (d, J=8.6Hz, 2H), 6.51 (dd, J=9.4, 3.6Hz, 1H), 5.04 (s, 2H), 4.83-4.68 (m, 2H), 4.27-4.15 (m, 2H).

### Embodiment 4: synthesis of benzylamine derivatives

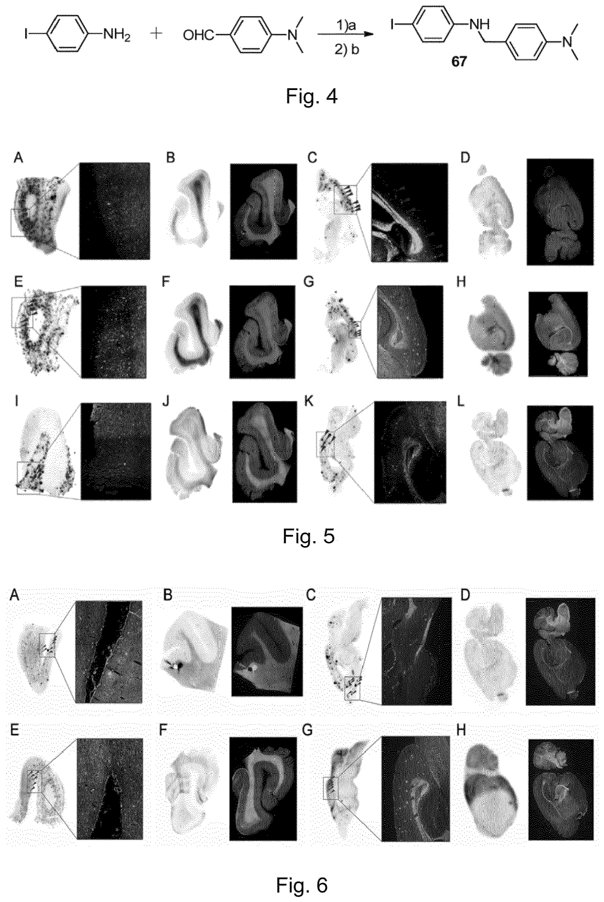

A synthesis reaction route is shown in Fig.4. Serial numbers of compounds in the embodiment 4 are consistent with serial numbers in the reaction route in Fig.4.

In the synthesis route shown in Fig.4, reagents and conditions are as follows: (a) EtOH, reflux; (b) NaBH₄, MeOH, reflux.

### 1) Synthesis of 4-(((4-iodophenyl)amino)methyl)-N,N-dimethylaniline (a compound 67)

4-iodoaniline (876.1mg, 4.0mmol) and 4-dimethylaminobenzaldehyde (596.8mg, 4.0mmol) are dissolved in 25ml of anhydrous ethanol, reaction is conducted for 15min at 90 °C under a reflux and stirring state, a great amount of white crystals are separated out, suction filtration is conducted after cooling, the crystals are washed with cold ethanol, the crystals are dried and then are dissolved in 50ml of methanol, NaBH₄ (453.6mg, 12.0mmol) is slowly added, and reaction is conducted for 30min at 90 °C under a reflux and stirring state. Reaction is monitor3ed through TLC till basic completion, depressurization is conducted to remove methanol, 50ml of deionized water is added, a great amount of white solid is separated out, suction filtration is conducted, the solid is washed with water and drying is conducted to obtain a white solid product (821.1mg, 58.3%). 1H NMR (400MHz, CDCl₃) δ 7.41 (d, J=8.5Hz, 2H), 7.22 (d, J=8.1Hz, 2H), 6.74 (s, 2H), 6.42 (d, J=8.6Hz, 2H), 4.17 (s, 2H), 2.95 (s, 6H).

### Embodiment 5: preparation of ¹²⁵I and ¹⁸F labeled ligands

### I. Experiment steps:

### 1) Preparation of compounds [¹²⁵I] 4, [¹²⁵I] 24, [¹²⁵I] 22, [¹²⁵I] 31a and [¹²⁵I] 35a

See Fig.1 for the synthesis reaction route. 0.1mg of each of corresponding tin precursors (respectively compounds 36, 37, 38, 39 and 40) is weighed and put in a glass reaction flask, 100µl of ethanol is added for dissolving, and then 1µl [¹²⁵] NaI solution ((200µCi, 2200Ci/mmol), 100µl of 1M hydrochloric acid and 50µl of H₂O₂ water solution (3%) are sequentially added. After sealing, reaction is conducted for 15min at room temperature, 20µl of saturated sodium hydrogen sulfite is added to terminate reaction, and a proper amount of NaHCO₃ is added to regulate pH to be neutral. Reaction liquid is separated through HPLC, separation conditions: Venusil MP C18 reversed phase column (5µm, 4.6x250mm); CH₃CN:H₂O=80%:20%; flow rate: 1.0ml/min. Separation conditions of [¹²⁵] 22: Venusil MP C18 reversed phase column (5µm, 4.6x250mm); CH₃CN:10mMAcNH₄=80%:20%; flow rate: 1.0ml/min. Retention time of ¹²⁵I labeled ligands and reference compounds is analyzed through HPLC.The obtained ¹²⁵I labeled ligands are stored at -20 °C for future use.

### 2) Preparation of compounds [¹⁸F] 44a, [¹⁸F] 53a and [¹⁸F] 53b

See Fig.2 for the synthesis reaction route. 1.0mg of each of corresponding labeled precursors (respectively compounds 48, 52a and 52b) is dissolved in 0.8ml of anhydrous acetonitrile to obtain solution, the solution is added into a dewatered reaction tube containing¹⁸F⁻ which has certain activity and contains K₂₂₂/K₂CO₃, labeling is conducted for 5min under the condition of 100 °C, and 10ml of deionized water is added after cooling to dilute reaction mixture. Mixed solution is purified through a pretreated Sep-Pak Plus C-18 solid phase extraction small column, then 10ml of deionized water is used to wash the column to remove unreacted [¹⁸F] F⁻ and inorganic salts, the column is dried by blowing N₂, then 2x1ml of anhydrous acetonitrile is used to elute labeled compounds and labeled precursors which are adsorbed onto the column, and separation and purification are conducted through HPLC after concentration, separation conditions: Venusil MP C18 reversed phase column (5µm, 10x250mm); CH₃CN:H₂O=70%:30%; flow rate: 4.0ml/min. Retention time of ¹⁸F labeled ligands and reference compounds is analyzed through HPLC under conditions which are the same as the separation conditions.

### II. Experiment results:

¹²⁵I labeled ligands are prepared through a classic tin-halogen exchange method. Labeling rates of [¹²⁵I] 4, [¹²⁵I] 24, [¹²⁵I] 22, [¹²⁵I] 31a and [¹²⁵I] 35a are sequentially 86.2%, 94.9%, 92.9%, 67.3% and 27.1%. After separation and purification are conducted through HPLC, radiochemical purity is higher than 95% and the retention time is consistent with the retention time of stable iodo ligands (see Table 1).

¹⁸F labeled compounds are prepared through a one-step method. Labelling rates of [¹⁸F] 44a, [¹⁸F] 53a and [¹⁸F] 53b are sequentially 13.8%, 13.4% and 23.9%. After separation and purification are conducted through HPLC, radiochemical purity is higher than 98% and the retention time is consistent with the retention time of stable ligands (see Table 1).

**Table 1: Retention time and purify of ¹²⁵I and ¹⁸F labeled ligands and stable ligands thereof**

| Compound | Flow rate (mL/min) | Flow phase (CH₃CN %) | Chromatographic column (Venusil MP C18) | Retention time (RT,min) | Purity (%) |
|---|---|---|---|---|---|
| 4 | 1 | 80 | 4.6X250 mm | 11.43 | 99.1% |
| [¹²⁵I]4 | 1 | 80 | 4.6X250 mm | 11.93 | 98.6% |
| 24 | 1 | 80 | 4.6X 250 mm | 10.93 | 99.5% |
| [¹²⁵1]24 | 1 | 80 | 4.6X250mm | 11.51 | 99.0% |
| 22 | 1 | 80 | 4.6X250 mm | 12.29 | 98.5% |
| [¹²⁵1]22 | 1 | 80 | 4.6X250 mm | 12.86 | 96.4% |
| 31a | I | 80 | 4.6X250 mm | 8.81 | 99.4% |
| [¹²⁵I]31a | I | 80 | 4.6X250 mm | 9.28 | 99.2% |
| | | | | | |
| 35a | 1 | 80 | 4.6X250 mm | S.2I | 99.7% |
| [¹²⁵I]35a | 1 | 80 | 4.6X 250 mm | 8.63 | 98.9% |
| 44a | 4 | 70 | 10X250 mm | 6.64 | 98.4% |
| C¹⁸F]44a | 4 | 70 | 10X250 mm | 6.77 | 99.2% |
| 53a | 4 | 70 | 10X250 mm | 9.14 | 95.4% |
| [¹⁸F] 53a | 4 | 70 | 10 X 250 mm | 9.25 | 98.4% |
| 53b | 4 | 70 | 10 X250 mm | 9.59 | 96.6% |
| C¹⁸F] 53b | 4 | 70 | 10 X250 mm | 9.72 | 98.8% |

### Experiment example 1: biological evaluaton

In-vitro competitive binding experiments (Ki determination) of compounds 4-25, 31a, 35a, 44a, 46, 47, 53a, 55, 57-59, 65 and 67, IMPY and PIB with Aβ₁₋₄₂ aggregates:
Binding reaction occurs between Aβ₁₋₄₂ aggregate proteins with certain concentration and radioactive ligands [¹²⁵I] IMPY, compounds (respectively prepared compounds 4-25, 31a, 35a, 44a, 46, 47, 53a, 55, 57-59, 65 and 67) with different concentration and to be determined, IMPY and PIB are simultaneously added into the reaction system to competitively react with [¹²⁵I] 4, the compounds are separated after balancing, and the inhibition constant (Ki) is calculated by determining radioactivity.

### 1. Experiment steps:

(1) Preparing 41 of PBS (0.2M) buffer solution with pH=7.4;
(2) Preparing radioligand [¹²⁵I] IMPY according to the existing method; preparing the [¹²⁵I] IMPY into 100000cpm/100µL water solution;
(3) Preparing the compounds to be determined into 10⁻³ to 10⁻⁹ mol/L continuously diluted ethanol solution;
(4) Preparing receptor Aβ₁₋₄₂ proteins according to the existing method and diluting the proteins into 30nM water solution;
(5) Soaking a glass fiber filter membrane in PBS solution containing 0.1% (volume fraction) polyethyleneimine for 0.5h;
(6) Respectively adding 100µl of solution of compounds with different concentration and to be determined, 100µl of [¹²⁵I] IMPY solution, 700µl of PBS and 100µl of Aβ₁₋₄₂ solution in a 12x75mm high borosilicate glass tube; sealing the tube by using a sealing film and whirling;
(7) Oscillating and incubating for 2h in 37 °C constant-temperature water bath;
(8) Collecting reaction liquid by using a multi-head cell collector, and washing the reaction liquid for three times by using PBS, 3ml per time;
(9) Measuring and counting by using a γ counter; and
(10) Processing data.

### 2. Experiment results

See Table 2 for half inhibition constants (IC₅₀) which are obtained through the competitive binding experiment and the inhibition constants Ki which are further calculated according to a formula.

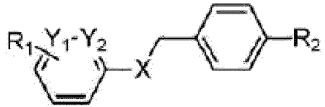

**Table 2: affinity constants of compounds 4-25, 31a, 35a, 44a, 46, 47, 53a and 53b with Aβ₁₋₄₂ aggregates**

| Compound | R₁ | R₂ | X | Y₁ | Y₂ | Ki(nM) |
|---|---|---|---|---|---|---|
| 4 | p-OMe | I | O | -CH- | -CH- | 28.7 ±8.0 |
| 5 | m-OMe | I | O | -CH- | -CH- | 154.5 ±5.6 |
| 6 | o-OMe | I | O | -CH- | -CH- | 6107 ±458 |
| 7 | p-OH | I | O | -CH- | -CH- | 134.0 ±28.1 |
| 8 | m-OH | I | O | -CH- | -CH- | 456.0 ± 135.0 |
| 9 | o-OH | I | O | -CH- | -CH- | 3348 ±611 |
| 10 | P-F | I | O | -CH- | -CH- | 126.6 ± 18.2 |
| 11 | p-Cl | I | O | -CH- | -CH- | 22.3 ± 2.6 |
| 12 | p-Br | I | O | -CH- | -CH- | 14.1 ±1.2 |
| 13 | p-I | I | O | -CH- | -CH- | 25.9 ±2.4 |
| 14 | p-H | I | O | -CH- | -CH- | 93.9 ±6.1 |
| 15 | p-Bu^{t} | I | O | -CH- | -CH- | 139.0 ±20.9 |
| 16 | p-NH₂ | I | O | -CH- | -CH- | 483.9±53.2 |
| 17 | m-NH₂ | I | O | -CH- | -CH- | 1815± 189 |
| 18 | o-NH₂ | I | O | -CH- | -CH- | 1216±58 |
| 19 | p-NHMe | I | O | -CH- | -CH- | 57.0 ±5.1 |
| 20 | m-NHMe | I | O | -CH- | -CH- | 701.9±92.4 |
| 21 | o-NHMe | I | O | -CH- | -CH- | > 20000 |
| 22 | p-NMe₂ | I | O | -CH- | -CH- | 20.9 ± 1.8 |
| 23 | m-NMe₂ | I | O | -CH- | -CH- | 1057± 104 |
| 24 | I | p-OMe | O | -CH- | -CH- | 58.5±3.5 |
| 25 | p-OMe | I | S | -CH- | -CH- | 626.9 ± 129.3 |
| 31a | p-OCH₂CH₂F | I | O | -CH- | -CH- | 24.1±8.8 |
| 35a | P-I | OCH₂CH₂F | O | -CH- | -CH- | 23.0 ± 3.3 |
| 44a | p-OMe | OCH₂CH₂F | O | -CH- | -CH- | 166.6±34.5 |
| 46 | p-NHMe | OCH₂CH₂F | O | -CH- | -CH- | 460.4 ± 82.0 |
| 47 | p-NMe₂ | OCH₂CH₂F | O | -CH- | -CH- | 23.0 ± 5.3 |
| 53a | P-OCH₂CH₂F | OMe | O | -CH- | -CH- | 464.9 ± 18.8 |
| 55 | p-Cl | I | O | N | -CH- | 879.8±72.4 |
| 57 | p-Br | I | O | N | -CH- | 163.5 ±23.0 |
| 58 | p-I | I | O | -CH- | N | 1060 ± 130.0 |
| 59 | p-I | I | O | N | -CH- | 824.0 ±61.6 |
| 65 | p-I | OCH₂CH₂F | O | N | -CH- | 2079±458 |
| 67 | p-I | NMe₂ | NH | -CH- | -CH- | 124.4 ± 14.3 |
| 1MPY | - | - | - | - | - | 38.1±2.5 |
| PIB | - | - | - | - | - | 45.9 ±3.1 |

According to the above-mentioned competitive binding experiments, it can be known that the compounds 4, 11, 12, 13, 22, 31a, 35a and 47 of the compounds described in the present invention have higher affinity with the Aβ₁₋₄₂ aggregates, and the affinity of the compounds is higher than that of the known compounds IMPY and PIB.

In this experiment example, the structural formulas of the known compounds IMPY and PIB are respectively:

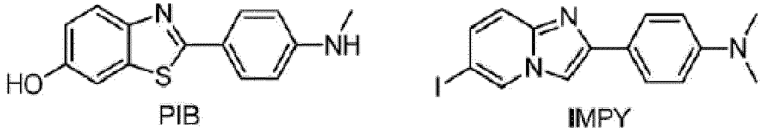

### Experiment example 2: autoradiography experiment

After ¹⁸F or ¹²⁵I labeled compounds with certain concentration are respectively bound with plaques in AD transgenic mouse and AD patient brain sections, exposure is conducted through a phosphor screen and then images are analyzed by using a storage phosphor screen system.

### 1. Experiment steps:

(1) Pretreating the AD transgenic mouse brain section and the AD patient brain section;
(2) Respectively covering the AD transgenic mouse brain section or the AD patient brain section with 100µl of 5µ Ci ¹⁸F or ¹²⁵I labeled compounds, and incubating for 60min at room temperature;
(3) Sequentially washing the section with lithium carbonate saturated 40% ethanol solution for 5min and then washing the section with flowing water for 5min; and
(4) After being air-dried, wrapping the section with a preservative film, placing the section under the phosphor screen for exposure for 120min and analyzing the images by using the storage phosphor screen system.

### 2. Experiment results:

Experiment results are shown in Fig.5 and Fig.6. It fully shows that, after the compounds of the present invention are labeled by radionuclide, the compounds can be used as brain Aβ plaque imaging agents, which can be applied to clinical diagnosis.

Fig.5 shows autoradiography results of [¹²⁵I] 4, [¹²⁵I] 24 and [¹²⁵I] 23 which are respectively applied to human brain sections ((A, E and I) AD, 64-year old, female; (B, F and J) normal, 74-year old, male) and mouse brain sections ((C, G and K) transgenic mice, APPswe/PSEN 1, 11-month old; (D, H and L) normal, C57BL6, 11-month old). Same sections are subjected to dyeing through thioflavin-S for contrast.

Fig.6 shows autoradiography results of [¹⁸F] 53a and [¹⁸F] 53b which are respectively applied to human brain sections ((A and E) AD, 64-year old, female; (B and F) normal, 74-year old, male) and mouse brain sections ((C and G) transgenic mice, APPswe/PSEN 1, 11-month old; (D and H) normal, C57BL6, 11-month old). Same sections are subjected to dyeing through thioflavin-S for contrast.

### Experiment example 3: experiments of biological distribution in bodies of normal mice

Through in-vivo distribution experiments, pharmacokinetic features, especially initial bran intake and brain removal situations of ¹⁸F or ¹²⁵I labeled compounds in the bodies of mice are studied.

### 1. Experiment steps

5-10µ Ci labeled compounds (100µl of normal saline solution, containing 5% ethanol) are injected into the bodies of normal mice (ICR, male, 20-22g, 5-week old) from caudal veins, the mice are beheaded respectively at the moment of 2min, 10min, 30min and 60min after injection, relevant organs are taken out through dissection, wet weight is measured and radioactive counting is conducted. Data are expressed in radioactive percentage dosage per organ (%ID/organ) and radioactive percentage dosage per gram of organ (%ID/g).

### 2. Experiment results

Experiment results are shown in Table 3. The ¹⁸F or ¹²⁵I labeled compounds of the present invention can smoothly pass through blood brain barriers, brain intake reaches a peak at the moment of 2min, the removing speed in the brains of normal mice is very fast and the ratio of brain intake at the moment of 2min to brain intake at the moment of 60min reaches approximate 10. Through further comparison with the known compounds such as [¹²⁵I] IMPY in the prior art, it can be found that the removing speed of the phenyl benzyl ether compounds in the brains of the normal mice is obviously superior to that of the known compounds.

In this experiment example, the structural formula of the known compound [¹²⁵I] IMPY is:

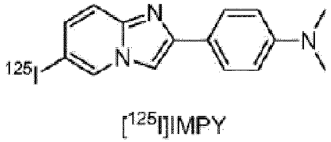

**Table 3: distribution results ^{a} of [¹²⁵I] 4, [¹²⁵I] 24, [¹²⁵I] 22, [¹²⁵I] 31a, [¹²⁵I] 35a, [¹²⁵I] IMPY, [¹⁸F] 44a, [¹⁸F] 53a and [¹⁸F] 53b in bodies of normal mice**

| Organ | 2min | 10 min | 30 min | 60 min |
|---|---|---|---|---|
| [¹²⁵I]4 (logD = 4.00± 0.08) | | | | |
| Blood | 3.03 ± 0.54 | 3.32 ± 0.65 | 2.56 ± 0.93 | 1.70 ± 0.50 |
| | | | | |
| Brain | 6.18 ± 0.99 | 3.54 ± 0.35 | 0.88 ± 0.15 | 0.38 ± 0.05 |
| Thyroid^{b} | 0.14 ± 0.04 | 0.12 ± 0.04 | 0.08 ± 0.01 | 0.11 ± 0.02 |
| [¹²⁵I]24 (logD = 4.16± 0.29) | | | | |
| Blood | 5.13 ± 0.62 | 4.26 ± 0.38 | 3.31 ± 0.79 | 2.60 ± 1.33 |
| Brain | 4.29 ± 1.06 | 2.11 ± 0.38 | 0.73 ± 0.17 | 0.32 ± 0.09 |
| Thyroid^{b} | 0.07 ± ().()1 | 0.07 ± 0.03 | 0.17 ± 0.03 | 0.19 ± 0.05 |
| [¹²⁵I]22(logD = 2.89± 0.09) | | | | |
| Blood | 4.01 ± 0.28 | 2.46 ± 0.30 | 2.53 ± 0.27 | 1.34 ± 0.26 |
| Brain | 4.91 ± 0.60 | 3.26 ± 0.24 | 1.34 ± 0.12 | 0.49 ± 0.07 |
| Thyroid^{b} | 0.11 ± 0.03 | 0.12 ± 0.03 | 0.16 ± 0.04 | 0.27 ±0.04 |
| [¹²⁵I]31a(logD = 3.96± 0.22) | | | | |
| Blood | 5.03 ± 0.20 | 3.03 ± 0.48 | 2.17 ± 0.31 | 1.% ± 0.31 |
| Brain | 7.04 ± 0.89 | 2.73 ± 0.25 | 1.05 ± 0.22 | 0.55 ± 0.11 |
| Thyroid^{b} | 0.12 ± 0.02 | 0.09 + 0.02 | 0.12 ± 0.01 | 0.16 ± 0.03 |
| [¹²⁵I]35a (logD = 2.50 ± 0.23) | | | | |
| Blood | 4.96 ± 0.35 | 3.88 ± 0.79 | 3.89 ± 0.53 | 3.01 ± 1.07 |
| Brain | 5.27 ± 0.98 | 2.28 ± 0.27 | 0.81 + 0.06 | 0.37 ± 0.06 |
| Thyroid^{b} | 0.13 ± 0.02 | 0.13 ± 0.01 | 0.19 ± 0.04 | 0.34 ± 0.10 |
| [¹²⁵I]IMPY | | | | |
| Blood | 3.85 ± 0.53 | 2.81 ± 0.33 | 1.50 ± 0.20 | 1.03 ± 0.10 |
| Brain | 5.05 ± 0.38 | 1.80 ± 0.12 | 0.67 ±0.15 | 0.45 ± 0.07 |
| Thyroid^{b} | 0.12 ± 0.05 | 0.09 ± 0.03 | 0.22 ± 0.08 | 0.30 ± 0.09 |
| [¹⁸F]44a(logD = 3.05 ± 0.08) | | | | |
| Blood | 3.90 ± 0.25 | 3.86 ± 0.24 | 4.28 ± 0.23 | 3.86 ± 0.39 |
| Brain | 7.54 ± 0.39 | 3.32 ±0.38 | 2.77 ±0.22 | 2.33 ±0.30 |
| Bone^{b} | 1.50 ± 0.44 | 1.04 ± 0.25 | 1.43 ± 0.44 | 4.05 ± 0.62 |
| [¹⁸F] 53a (logD= 3.65 ±0.04) | | | | |
| Blood | 4.26 ± 0.41 | 4,61 ± 0.95 | 3.76 ± 0.19 | 3.05 ±1.09 |
| Brain | 9.95 ± 2.36 | 2.77 + 0.19 | 1.44 ± 0.18 | 1.30 ± 0.16 |
| Bone^{b} | 2.37 ± 0.69 | 2.76 ± 0.47 | 4.02 ± 0.78 | 5.89 ± 0.49 |
| [¹⁸F] 53b (logD = 3.04 ± 0.04) | | | | |
| Blood | 3.95 ± 0.34 | 4.14 ± 0.91 | 3.40 ± 0.25 | 2.36 ± 0.66 |
| Brain | 9.71 ± 1.31 | 2.52 ± 0.20 | 1.39 ± 0.10 | 1.07 ± 0.10 |
| Bone^{b} | 1.36 ± 0.35 | 1.22 ± 0.42 | 1.39 ± 0.54 | 1.52 ± 0.27 |

| | | | | |
|---|---|---|---|---|
| ^{a}is expressed in % ID/g, n=4-5. ^{b}is expressed in % ID/organ. | | | | |

Although the present invention has been described above in details by using general description and specific embodiments, some modifications or improvements can be made on the basis of the present invention and it is obvious for one skilled in the art. Therefore, any modifications or improvements made without departing from the spirit of the present invention belong to the protection scope claimed by the present invention.

## Claims

1. A phenyl benzyl ether derivative having a structural formula shown by a formula (I): 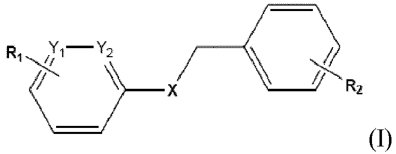
wherein X is O, NH or S; Y₁ and Y₂ respectively and independently denote - CH- or nitrogen;
R₁ and R₂ respectively and independently denote nitrogen, halogen, hydroxyl, hydrosulfuryl, alkoxy, alkyl, carbocyclic alkyl, heterocyclic alkyl, nitro, amino, alkylamino, cyan, carboxyl, aryl, heteraryl, arylalkoxy, substituted arylalkoxy, aryloxy, substituted aryloxy, arylalkenyl, substituted arylalkenyl, -O (CH₂)mNRaRb, -CO-NRaRb, -NHCO-Ra, -Sn (alkyl)₃, -(CH₂)m-Z, -O (CH₂)m-Z, -(CH₂)m- aryl or -(OCH₂CH₂)n-Z;
Ra and Rb respectively and independently denote hydrogen, akyl or -(CH₂)m-aryl; Z denotes halogen, hydroxyl, trifluoromethylsulfonyl, methylsulfonyl or tolylsulfonyl; m and n are respectively integers from 1 to 6, preferably integers from 1 to 3 respectively.

2. The phenyl benzyl ether derivative according to claim 1, wherein R₁ and R₂ are o-substituents, m-substituents or p-substituents.

3. The phenyl benzyl ether derivative according to claim 1 or claim 2, wherein the halogen is fluorine, chlorine, bromine or iodine; the alkoxy is C₁-C₁₂ alkoxy, preferably C₁-C₆ alkoxy; the alkyl is C₁-C₁₂ alkyl, preferably C₁-C₆ alkyl; the carbocyclic alkyl is three-membered to six-membered carbocyclic alkyl, preferably cyclopropyl, cyclopentyl or cyclohexyl; heterocyclic alkyl is three-membered to six-membered heterocyclic alkyl, preferably piperidyl, piperazinyl or morpholinecyclic group; the alkylamino is C₁-C₁₂ alkylamino, preferably C₁-C₆ alkylamino, more preferably N-methylamino, dimethylamino, diethylamino, dipropylamino or diisopropylamino; the aryl is phenyl or naphthyl; the heteraryl is pyridyl, furyl, thienyl, benzothiazolyl, benzofuryl or benzoxazolyl; the arylalkoxy is C₅-C₇ aryl C₁-C₁₂ alkoxy, preferably phenylmethoxyl or phenylethyoxyl; the substituted arylalkoxy is substituted C₅-C₇ aryl C₁-C₁₂ alkoxy, preferably substituted phenylmethoxyl or substituted phenylethyoxyl; the aryloxy is C₅-C₇ aryloxy, preferably cyclopentadienyloxy or phenyloxy; the substituted aryloxy is substituted C₅-C₇ aryloxy, preferably substituted cyclopentadienyloxy or substituted phenyloxy; the arylalkenyl is C₅-C₇ aryl C₂-C₆ alkenyl, preferably phenylvinyl; and the substituted arylalkenyl is substituted C₅-C₇ aryl C₂-C₆ alkenyl, preferably substituted phenylvinyl.

4. The phenyl benzyl ether derivative according to claim 1, 2 or 3, wherein the structural formula is shown by a formula (I-1): 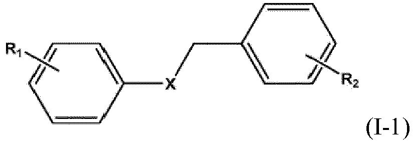
wherein X is O, NH or S;
R₁ is nitro, methoxy, hydroxyl, fluorine, chorine, bromine, iodine, hydrogen, tert-butyl, amino, methylamino, dimethylamino, -OCH₂CH₂F, -Sn(butyl)₃, -OCH₂CH₂OH, -(OCH₂CH₂)₃OH, -OCH₂CH₂OTs, -(OCH₂CH₂)₃OTs or -(OCH₂CH₂)₃F;
R₂ is iodine, methoxy, bromine, hydrogen, -OCH₂CH₂F, -Sn(butyl)₃,-OCH₂CH₂Br, -OCH₂CH₂OTs or dimethylamino; preferably, R₁ and R₂ are respectively:
| R₁ | R₂ |
|---|---|
| Nitro | Iodine |
| Methoxy | Iodine |
| Hydroxyl | Iodine |
| Fluorine | Iodine |
| Chlorine | Iodine |
| Bromine | Iodine |
| Iodine | Iodine |
| Hydrogen | Iodine |
| Tert-butyl | Iodine |
| Amino | Iodine |
| Methylamino | Iodine |
| Dimethylamino | Iodine |
| Iodine | Methoxy |
| Methoxy | Bromine |
| Bromine | Methoxy |
| Dimethylamino | Bromine |
| -OCH₂CH₂F | Hydrogen |
| -OCH₂CH₂F | Iodine |
| -OCH₂CH₂F | Bromine |
| Iodine | -OCH₂CH₂F |
| Bromine | -OCH₂CH₂F |
| Methoxy | -Sn(butyl)₃ |
| -Sn(butyl)₃ | Methoxy |
| Dimethylamino | -Sn(butyl)₃ |
| -OCH₂CH₂F | -Sn(butyl)₃ |
| -Sn(butyl)₃ | -OCH₂CH₂F |
| Methoxy | -OCH₂CH₂F |
| Methoxy | -OCH₂CH₂Br |
| Nitro | -OCH₂CH₂F |
| Amino | -OCH₂CH₂F |
| Methylamino | -OCH₂CH₂F |
| Dimethylamino | -OCH₂CH₂F |
| Methoxy | -OCH₂CH₂OTs |
| -OCH₂CH₂OH | Hydrogen |
| -(OCH₂CH₂)₃OH | Hydrogen |
| -OCH₂CH₂OH | Methoxy |
| -(OCH₂CH₂)₃OH | Methoxy |
| -OCH₂CH₂OTs | Methoxy |
| -(OCH₂CH₂)₃OTs | Methoxy |
| -OCH₂CH₂F | Methoxy |
| -(OCH₂CH₂)₃F | Methoxy |
| Iodine | Dimethylamino |
and more preferably, R₁, R₂ and X are respectively:
| R₁ | R₂ | X |
|---|---|---|
| p-OMe | I | O |
| m-OMe | I | O |
| o-OMe | I | O |
| p-OH | I | O |
| m-OH | I | O |
| o-OH | I | O |
| p-F | I | O |
| p-Cl | I | O |
| p-Br | I | O |
| p-I | I | O |
| P-H | I | O |
| p-Bu^{t} | I | O |
| p-NH₂ | I | O |
| m-NH₂ | I | O |
| o-NH₂ | I | O |
| p-NHMe | I | O |
| m-NHMe | I | O |
| o-NHMe | I | O |
| p-NMe₂ | I | O |
| m-NMe2 | I | O |
| I | P-OMe | O |
| p-OMe | I | S |
| p-OCH₂CH₂F | I | O |
| p-I | OCH₂CH₂F | O |
| p-OMe | OCH₂CH₂F | O |
| p-NHMe | OCH₂CH₂F | O |
| p-NMe₂ | OCH₂CH₂F | O |
| p-OCH₂CH₂F | OMe | O |
| P-(OCH₂CH₂)₃F | OMe | O |
| p-I | NMe₂ | NH |

5. The phenyl benzyl ether derivative according to claim 1, 2 or 3, wherein the structural formula is shown by a formula (I-2):
wherein X is O, NH or S;
R₁ is hydrogen, bromine, iodine, nitro, amino, methylamino or dimethylamino;
R₂ is iodine, methoxy or -OCH₂CH₂F;
preferably, R₁ and R₂ are respectively:
| R₁ | R₂ |
|---|---|
| Hydrogen | Iodine |
| Bromine | Iodine |
| Iodine | Iodine |
| Nitro | Iodine |
| Amino | Iodine |
| Methylamino | Iodine |
| Dimethylamino | Iodine |
| Iodine | Methoxy |
| Iodine | -OCH₂CH₂F |
and more preferably, R₁, R₂ and X are respectively:
| R₁ | R₂ | X |
|---|---|---|
| p-H | I | O |
| p-I | I | O |

6. The phenyl benzyl ether derivative according to claim 1, 2 or 3, wherein the structural formula is shown by a formula (I-3): 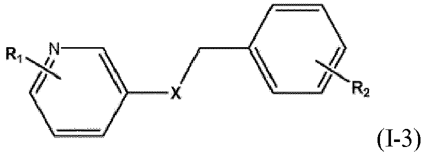
wherein X is O, NH or S; R₁ is chlorine, bromine or iodine; R₂ is iodine, methoxy or -OCH₂CH₂;
preferably, R₁ and R₂ are respectively:
| R₁ | R₂ |
|---|---|
| Chlorine | Iodine |
| Bromine | Iodine |
| Iodine | Iodine |
| Iodine | Methoxy |
| Iodine | -OCH₂CH₂F |
and more preferably, R₁, R₂ and X are respectively:
| R₁ | R₂ | X |
|---|---|---|
| p-Cl | I | O |
| p-Br | I | O |
| p-I | I | O |
| p-I | OCH₂CH₂F | O |

7. The phenyl benzyl ether derivative according to claim 1, 2, 3, 4, 5 or 6, wherein when the phenyl benzyl ether derivative contains fluorine atoms, F is ¹⁸F or ¹⁹F; when the phenyl benzyl ether derivative contains iodine atoms, I is ¹²³I, ¹²⁴I, ¹²⁵I, ¹²⁷I or ¹³¹I; and when the phenyl benzyl ether derivative contains methyl, methoxy, N-methylamino or dimethylamino, -CH₃ is -¹¹CH3, -OCH₃ is -O¹¹CH₃, -NHCH₃ is -NH¹¹CH₃, and -N(CH₃)₂ is -N(¹¹CH₃)₂ or -N(CH₃)(¹¹CH₃).

8. A preparation method of a phenyl benzyl ether derivative according to claim 1, 2 or 3, wherein a reaction equation is: 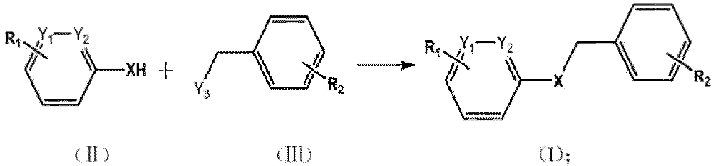 wherein Y₃ is bromine or chlorine; and R₁, R₂, X, Y₁ and Y₂ are correspondingly defined as that in the phenyl benzyl ether derivative shown by a structural formula such as a formula (I).

9. A preparation method of a phenyl benzyl ether derivative according to claim 4, wherein a reaction equation is: 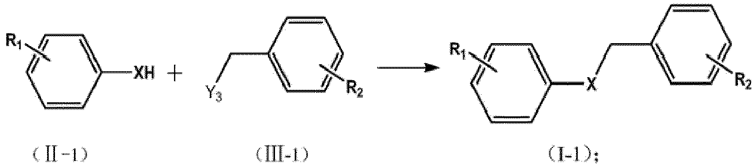 wherein Y₃ is bromine or chlorine; R₁, R₂ and X are correspondingly defined as that in the phenyl benzyl ether derivative shown by a structural formula such as a formula (I-1).

10. A preparation method of a phenyl benzyl ether derivative according to claim 5, wherein a reaction equation is: 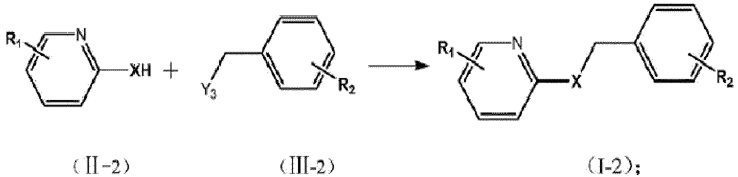 wherein Y₃ is bromine or chlorine; R₁, R₂ and X are correspondingly defined as that in the phenyl benzyl ether derivative shown by a structural formula such as a formula (I-2).

11. A preparation method of a phenyl benzyl ether derivative according to claim 6, wherein a reaction equation is: 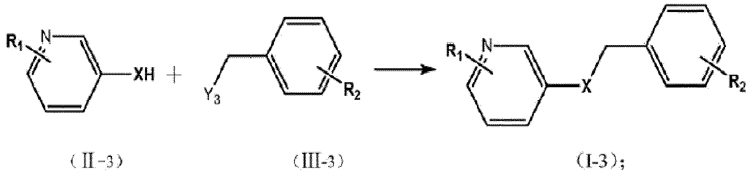 wherein Y₃ is bromine or chlorine; R₁, R₂ and X are correspondingly defined as that in the phenyl benzyl ether derivative shown by a structural formula such as a formula (I-3).

12. An Aβ plaque imaging agent prepared by using a phenyl benzyl ether derivative according to claim 1, 2, 3, 4, 5, 6 or 7,
wherein, when the phenyl benzyl ether derivative contains fluorine atoms, prepared compounds containing F-18 are used as the Aβ plaque imaging agent, especially a positron emission tomography (PET) Aβ plaque imaging agent;
or when the phenyl benzyl ether derivative contains iodine atoms, prepared compounds containing I-124 are used as the Aβ plaque imaging agent, especially the PET Aβ plaque imaging agent;
or when the phenyl benzyl ether derivative contains methyl, methoxy, N-methylamino or dimethylamino, prepared compounds containing C-11 are used as the Aβ plaque imaging agent, especially the PET Aβ plaque imaging agent;
or when the phenyl benzyl ether derivative contains iodine atoms, prepared compounds containing I-123, I-125 or I-131 are used as the Aβ plaque imaging agent, especially a single photon emission computed tomography (SPECT) Aβ plaque imaging agent.

13. The Aβ plaque imaging agent according to claim 12, wherein the Aβ plaque imaging agent is a single photon or positron Aβ plaque imaging agent.

14. A method for preparation of a medicine for diagnosing an amyloidosis disease, comprising applying an Aβ plaque imaging agent of claim 12 or claim 13.

15. A method for preparation of a medicine for diagnosing and treating an Alzheimer's disease, comprising applying a phenyl benzyl ether derivative of claim 1, 2, 3, 4, 5, 6 or 7.
